(19) Europäisches Patentamt
European Patent Office
Office européen des brevets

(11) Veröffentlichungsnummer : **0 137 456**
**B1**

(12) # EUROPÄISCHE PATENTSCHRIFT

(45) Veröffentlichungstag der Patentschrift : **10.02.88**

(21) Anmeldenummer : **84111909.2**

(22) Anmeldetag : **04.10.84**

(51) Int. Cl.⁴ : **C 07 D213/30**, C 07 D213/26, C 07 D241/12, C 07 D213/89, C 07 D239/26, A 01 N 43/40, A 01 N 43/60, A 01 N 43/54

(54) **Pyridin-, Pyrazin- und Pyrimidinderivate und deren Verwendung als fungizide Wirkstoffe.**

(30) Priorität : **07.10.83 CH 5458/83**
**26.07.84 CH 3620/84**

(43) Veröffentlichungstag der Anmeldung :
**17.04.85 Patentblatt 85/16**

(45) Bekanntmachung des Hinweises auf die Patenterteilung : **10.02.88 Patentblatt 88/06**

(84) Benannte Vertragsstaaten :
**AT BE CH DE FR GB IT LI NL SE**

(56) Entgegenhaltungen :
**EP-A- 0 069 330**
**EP-A- 0 074 018**
**DE-A- 3 150 138**
**GB-A- 1 496 431**
**Die Akte enthält technische Angaben, die nach dem Eingang der Anmeldung eingereicht wurden und die nicht in dieser Patentschrift enthalten sind.**

(73) Patentinhaber : **F. HOFFMANN-LA ROCHE & CO. Aktiengesellschaft**
**CH-4002 Basel (CH)**

(72) Erfinder : **Dorn, Franz, Dr.**
**Bohnackerstrasse 5**
**CH-8157 Dielsdorf (DE)**
Erfinder : **Pfiffner, Albert, Dr.**
**Grampenweg 10**
**CH-8180 Bülach (CH)**
Erfinder : **Zehnder, Beat, Dr.**
**Liebrütistrasse 40**
**CH-4303 Kaiseraugst (CH)**

(74) Vertreter : **Lederer, Franz, Dr. et al**
**Van der Werth, Lederer & Riederer Patentanwälte**
**Lucile-Grahn-Strasse 22**
**D-8000 München 80 (DE)**

**EP 0 137 456 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft heterocyclische Verbindungen, und zwar Pyridin-, Pyrazin- und Pyrimidinderivate der allgemeinen Formel

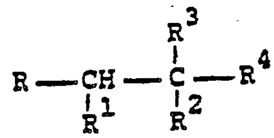

(I)

worin

R mono-, di- oder trisubstituiertes Phenyl, wobei die Substituenten 1-3 Halogenatome und/oder 1 oder 2 $C_{1-3}$-Alkylgruppen und/oder 1 oder 2 $C_{1-3}$-Alkoxygruppen und/oder 1 oder 2 Trifluormethylgruppen sind.

$R^1$ gegebenenfalls mit einer $C_{1-4}$-Alkoxygruppe substituiertes $C_{1-6}$-Alkyl, $C_{3-6}$-Alkenyl, $C_{3-6}$-Alkinyl, $C_{1-6}$-Alkylthio, $C_{3-6}$-Alkenylthio oder $C_{3-6}$-Alkinylthio ; oder gegebenenfalls mono-, di- oder trisubstituiertes Arylthio, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind,

$R^2$ Hydroxy, Chlor oder Brom,

$R^3$ $C_{2-6}$-Alkenyl ; gegebenenfalls im Arylkern mono-, di- oder trisubstituiertes Aryl oder Aryl-$C_{1-3}$-alkyl, wobei die gegebenenfalls vorhandenen Substituenten 1-3 Halogenatome und/oder 1 oder 2 $C_{1-3}$-Alkylgruppen und/oder 1 oder 2 $C_{1-3}$-Alkoxygruppen und/oder eine Nitrogruppe sind ; oder eine Gruppe $C{\equiv}CR^5$,

$R^4$ 3-Pyridyl, 3-Pyridyl-1-oxid, 2-Pyrazinyl, 2-Pyrazinyl-1-oxid, 2-Pyrazinyl-4-oxid, 2-Pyrazinyl-1,4-dioxid, 5-Pyrimidinyl, 5-Pyrimidinyl-1-oxid oder 5-Pyrimidinyl-1,3-dioxid und

$R^5$ Wasserstoff ; gegebenenfalls mit 1 oder 2 $C_{1-3}$-Alkoxygruppen, einem Chloratom oder einem Bromatom substituiertes $C_{1-10}$-Alkyl ; Vinyl ; $C_{1-6}$-Alkoxy ; Brom ; Jod ; oder gegebenenfalls mono-, di- oder trisubstituiertes Aryl, wobei die gegebenenfalls vorhandenen Substituenten 1-3 Halogenatome und/oder 1 oder 2 $C_{1-3}$-Alkylgruppen und/oder 1 oder 2 $C_{1-3}$-Alkoxygruppen sind, bedeuten,

und Säureadditionssalze dieser Verbindungen.

Die Verbindungen der Formel I und deren Säureadditionssalze besitzen fungizide Eigenschaften und eignen sich als fungizide Wirkstoffe, insbesondere zur Verwendung in der Landwirtschaft und im Gartenbau.

Die Erfindung betrifft ferner ein Verfahren zur Herstellung der Verbindungen der Formel I und ihrer Säureadditionssalze, Verbindungen der Formel I und Säureadditionssalze davon als fungizide Wirkstoffe, fungizide Mittel, die Verbindungen der Formel I oder Säureadditionssalze davon als Wirkstoffe enthalten, sowie die Verwendung solcher Verbindungen, Säureadditionssalze und Mittel zur Bekämpfung von Fungi in der Landwirtschaft und im Gartenbau.

Die europäische Patentpublikation Nr. 69 330 beschreibt u. a. fungizid wirksame aromatisch substituierte α-Alkenyl-α-benzyl-3-pyridylmethanole sowie deren pflanzenphysiologisch verträgliche Säureadditionssalze. Die erfindungsgemässen Verbindungen unterscheiden sich von den bekannten Verbindungen im wesentlichen durch die Substitution der dem Benzylrest gehörenden α-Methylenbrücke mit einem Rest $R^1$ : die bekannten Verbindungen weisen ausnahmslos eine unsubstituierte Methylenbrücke auf.

Die europäische Patentpublikation Nr. 74.018 beschreibt u. a. fungizid wirksame aromatisch substituierte α-(α-Alkyl- oder α-Alkylthiobenzyl)-3-pyridylmethanole und -2-pyrazinylmethanole und deren N-Oxide und Säureadditionssalze sowie die entsprechenden Methylchloride. Diese Verbindungen können auch noch einen α-Alkylsubstituenten aufweisen. Die erfindungsgemässen Verbindungen unterscheiden sich von den bekannten Verbindungen im wesentlichen durch die zwingende Anwesenheit einer α-Alkenyl-, α-Aryl-, α-Arylalkyl- oder α-Alkinylgruppe $R^3$ anstelle des obenerwähnten fakultativen α-Alkylsubstituenten.

In der obigen Formel I umfasst der Ausdruck « Halogen » Fluor, Chlor, Brom und Jod. Die Alkyl-, Alkenyl- und Alkinylgruppen können geradkettig oder verzweigt sein, wobei dies auch für den Alkyl-, Alkenyl- oder Alkinylteil der Alkoxy-, Arylalkyl-, Alkylthio-, Alkenylthio- und Alkinylthiogruppen gilt. Der Ausdruck « Aryl » an sich oder als Teil von « Arylthio » oder « Aryl-$C_{1-3}$-alkyl » bedeutet vorzugsweise Phenyl, wobei aber auch heterocyclische Gruppen mit aromatischem Charakter, wie Pyridyl, Furyl und Thienyl, in Betracht kommen. In di- oder trisubstituiertem Phenyl, Arylthio, Aryl, Arylalkyl oder Alkyl können die Substituenten gleich oder verschieden sein.

Eine interessante Untergruppe von Verbindungen der Formel I besteht aus denjenigen Verbindungen der Formel I, worin R Mono-, Di- oder Trihalophenyl ; $R^1$ unsubstituiertes $C_{1-6}$-Alkyl, $C_{3-6}$-Alkenyl, $C_{3-6}$-Alkinyl oder $C_{1-6}$-Alkylthio ; $R^2$ Hydroxy oder Chlor ; $R^3$ $C_{2-6}$-Alkenyl, gegebenenfalls substituiertes Aryl, wie oben näher definiert, oder eine Gruppe $C{\equiv}CR^5$, worin $R^5$ Wasserstoff, gegebenenfalls substituiertes $C_{1-10}$-Alkyl, wie oben näher definiert, $C_{1-6}$-Alkoxy, Brom, Jod oder gegebenenfalls substituiertes Aryl,

wie oben näher definiert, darstellt ; und $R^4$ 3-Pyridyl, 2-Pyrazinyl oder 5-Pyrimidinyl bedeuten.

Unabhängig voneinander bedeuten R vorzugsweise Mono-, Di- oder Trihalophenyl, insbesondere 2,4-Dichlorphenyl, und $R^4$ vorzugsweise 3-Pyridyl, 2-Pyrazinyl oder 5-Pyrimidinyl, insbesondere 3-Pyridyl oder 2-Pyrazinyl.

Besonders bevorzugte Verbindungen der Formel I sind :

α-Aethinyl-α-(2,4-dichlor-α-methylbenzyl)-3-pyridylmethanol,
α-(2,4-Dichlor-α-methylbenzyl)-α-(1-pentinyl)-3-pyridylmethanol,
α-(2,4-Dichlor-α-methylbenzyl)-α-jodäthinyl-3-pyridylmethanol,
α-(2,4-Dichlor-α-methylbenzyl)-α-vinyl-3-pyridyl-methanol,
α-Aethinyl-α-(2,4-dichlor-α-methylthio-benzyl)-3-pyridylmethanol,
α-Aethoxyäthinyl-α-(2,4-dichlor-α-methylbenzyl)-3-pyridylmethanol,
α-Aethinyl-α-[2,4-dichlor-α-(2-propinyl)-benzyl]-3-pyridylmethanol,
α-(2,4-Dichlor-α-methylbenzyl)-α-(1-propen-1-yl)-3-pyridylmethanol,
α-(2,4-Dichlor-α-methylbenzyl)-α-isopropenyl-3-pyridylmethanol und
α-(2,4-Dichlor-α-methylbenzyl)-α-[(Z)-1-pentenyl]-3-pyridylmethanol, also die Verbindungen der Formel

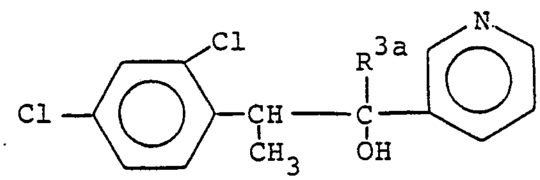

(Ia)

worin $R^{3a}$ Vinyl, 1-Propen-1-yl, Isopropenyl, Aethinyl, 1-Pentinyl, Aethoxyäthinyl oder Jodäthinyl, sowie α-Aethinyl-α-(2,4-dichlor-α-methylthio-benzyl)-3-pyridylmethanol,
α-Aethinyl-α-[2,4-dichlor-α-(2-propinyl)-benzyl]-3-pyridylmethanol und
α-(2,4-Dichlor-α-methylbenzyl)-α-[(Z)-1-pentenyl]-3-pyridylmethanol.

Weitere Vertreter von Verbindungen der Formel I sind :

α-Aethinyl-α-(2,4-dichlor-α-methylbenzyl)-5-pyrimidylmethanol,
α-Aethinyl-α-(2,4-dichlor-α-methylbenzyl)-2-pyrazinylmethanol,
α-(But-3-en-1-inyl)-α-(2,4-dichlor-α-methylbenzyl)-3-pyridylmethanol,
α-(2,4-Dichlor-α-methylbenzyl)-α-vinyl-5-pyrimidinylmethanol und
α-(2,4-Dichlor-α-methylbenzyl)-α-vinyl-2-pyrazinylmethanol.

Das Vorhandensein mindestens eines asymmetrischen Kohlenstoffatoms in den Verbindungen der Formel I hat zur Folge, dass die Verbindungen in isomeren Formen auftreten können. Durch das Vorliegen einer allfälligen aliphatischen C=C-Doppelbindung kann auch geometrische Isomerie auftreten. Die Formel I soll all diese möglichen isomeren Formen umfassen.

Als Säureadditionssalze der Verbindungen der Formel I kommen physiologisch verträgliche Salze in Frage. Hierzu gehören vorzugsweise Salze dieser Verbindungen mit anorganischen und organischen Säuren, wie Salzsäure ; Salpetersäure ; Phosphorsäure ; mono- und bifunktionellen Carbonsäuren und Hydroxycarbonsäuren, z. B. Essigsäure, Maleinsäure, Bernsteinsäure, Fumarsäure, Weinsäure, Zitronensäure, Salicylsäure, Sorbinsäure und Milchsäure ; und Sulfonsäuren, z. B. 1,5-Naphthalin-disulfonsäure. Im Falle der N-Oxide, d. h. der Verbindungen der Formel I, worin $R^2$ 3-Pyridyl-1-oxid, 2-Pyrazinyl-1-oxid, -4-oxid und -1,4-dioxid, und 5-Pyrimidinyl-1-oxid und -1,3-dioxid, kommen als Säureadditionssalze insbesondere physiologisch verträgliche Salze mit starken Säuren in Frage, wie anorganische Säuren, z. B. Salzsäure, Salpetersäure und Phosphorsäure, und Sulfonsäuren, z. B. 1,5-Naphthalindisulfonsäure.

Das erfindungsgemässe Verfahren zur Herstellung der Verbindungen der Formel I und von ihren Säureadditionssalzen ist dadurch gekennzeichnet, dass man

a) zwecks Herstellung der Verbindungen der Formel I, in der $R^2$ Hydroxy und $R^4$ 3-Pyridyl, 2-Pyrazinyl oder 5-Pyrimidinyl bedeuten und $R^3$ — mit Ausnahme von C≡CBr oder C≡CJ — die obige Bedeutung besitzt, ein Keton der allgemeinen Formel

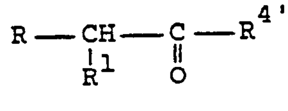

(II)

worin

R und $R^1$ die oben angegebenen Bedeutungen besitzen und
$R^{4'}$ 3-Pyridyl, 2-Pyrazinyl oder 5-Pyrimidinyl bedeutet,

mit einer Verbindung der allgemeinen Formel

$$(R^{3'})_nX \qquad (III)$$

worin

$R^{3'}$ die Bedeutung von $R^3$, mit Ausnahme von C≡CBr oder C≡CJ, besitzt und

X ein Alkalimetall, z. B. Lithium, Natrium oder Kalium, ein Erdalkalimetall, z. B. Calcium oder Magnesium, oder MgHal,

n die Wertigkeit von X und

Hal Halogen, insbesondere Brom oder Jod, bedeuten,

umsetzt.

b) zwecks Herstellung der Verbindungen der Formel I, in der $R^2$ Hydroxy, $R^3$ die Gruppe C≡CBr oder C≡CJ und $R^4$ 3-Pyridyl, 2-Pyrazinyl oder 5-Pyrimidinyl bedeuten, ein Aethinderivat der allgemeinen Formel

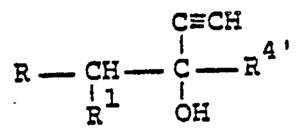

(I')

worin R, $R^1$ und $R^{4'}$ die oben angegebenen Bedeutungen besitzen, mit einem Bromierungsmittel bzw. Jodierungsmittel behandelt,

c) zwecks Herstellung der Verbindungen der Formel I, worin

$R^2$ Chlor oder Brom und $R^4$ 3-Pyridyl, 2-Pyrazinyl oder 5-Pyrimidinyl bedeuten, einen Alkohol der allgemeinen Formel

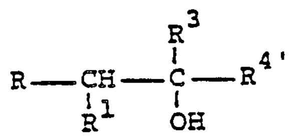

(I'')

worin R, $R^1$, $R^3$ und $R^{4'}$ die oben angegebenen Bedeutungen besitzen, mit einem Chlorierungsmittel bzw. Bromierungsmittel behandelt,

d) zwecks Herstellung der Verbindungen der Formel I, worin $R^3$ 1-$C_{2-6}$-Alkenyl und $R^4$ 3-Pyridyl, 2-Pyrazinyl oder 5-Pyrimidinyl bedeuten, ein Alkinderivat der allgemeinen Formel

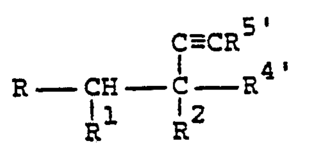

(I''')

worin

R, $R^1$, $R^2$ und $R^{4'}$ die oben angegebenen Bedeutungen besitzen und

$R^{5'}$ Wasserstoff oder $C_{1-4}$-Alkyl bedeutet, reduziert, oder

e) zwecks Herstellung der Verbindungen der Formel I, worin $R^4$ 3-Pyridyl-1-oxid, 2-Pyrazinyl-1-oxid, 2-Pyrazinyl-4-oxid, 2-Pyrazinyl-1,4-dioxid, 5-Pyrimidinyl-1-oxid oder 5-Pyrimidinyl-1,3-dioxid bedeutet, ein Pyridin- Pyrazin- bzw. Pyrimidinderivat der allgemeinen Formel

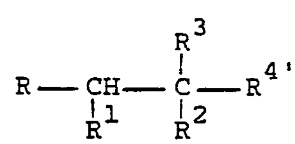

(I'''')

worin R, $R^1$, $R^2$, $R^3$ und $R^{4'}$ die oben angegebenen Bedeutungen besitzen, N-oxydiert und erwünschtenfalls eine erhaltene Verbindung der Formel I durch Umsetzung mit einer Säure in das entsprechende Säureadditionssalz überführt.

Die Verfahrensvariante a) wird zweckmässigerweise durchgeführt, indem man das Keton der Formel II mit der Verbindung der Formel III in einem inerten Verdünnungsmittel, vorzugsweise einem aprotischen Lösungsmittel, wie einem aliphatischen oder cyclischen Aether, z. B. Diäthyläther, Tetrahydrofuran oder Dioxan, und in einem Temperaturbereeich zwischen —80 °C und der Rückflusstemperatur des Reaktionsgemisches, vorzugsweise zwischen —80 °C und 0 °C (im Falle von X = Lithium) bzw. zwischen 0 °C und der Rückflusstemperatur (im Falle von X = MgHal), umsetzt. In vielen Fällen erweist es sich als vorteilhaft, die Verbindung der Formel III im Ueberschuss zu verwenden, und zwar bis zu 200 Molprozenten. In der Formel III ist X vorzugsweise Lithium oder MgHal.

Bei der Verfahrensvariante b) wird die Umsetzung zweckmässigerweise dadurch durchgeführt, dass man das Aethinderivat der Formel I' in einem inerten Verdünnungsmittel in Gegenwart einer Base mit dem Bromierungsmittel bzw. Jodierungsmittel, vorzugsweise elementarem Brom bzw. Jod, behandelt. Die Reaktionstemperaturen liegen je nach Art der Base zwischen —80 °C und der Rückflusstemperatur des Reaktionsgemisches. Das Verdünnungsmittel ist vorzugsweise ein organisches Lösungsmittel, wie ein aliphatischer oder aromatischer Kohlenwasserstoff, z. B. n-Hexan, Benzol, Toluol oder ein Xylol; ein halogenierter aromatischer Kohlenwasserstoff, z. B. Chlorbenzol; ein tertiäres Amin, z. B. Triäthylamin oder Pyridin; ein aliphatischer oder araliphatischer Alkohol, z. B. Methanol, Aethanol, n-Propanol, Isopropanol oder Benzylalkohol; oder ein aliphatischer oder cyclischer Aether, z. B. Diäthyläther, Butylmethyläther, Dioxan oder Tetrahydrofuran. Bevorzugte Basen sind Alkalimetallhydroxide, z. B. Natriumhydroxid; Alkalimetall- und Erdalkalimetallhydride, z. B. Natriumhydrid; und Organolithium- und Organomagnesiumverbindungen.

Die Chlorierung nach Verfahrensvariante c) wird zweckmässigerweise, wie in der europäischen Patentpublikation Nr. 74018, Seite 9, Zeilen 1-20, für eine analoge Chlorierung beschrieben, durchgeführt. Im Falle der Bromierung wird als Bromierungsmittel vorzugsweise Phosphortribromid verwendet. Die Bromierung wird prinzipiell unter denselben Reaktionsbedingungen wie für die Chlorierung durchgeführt. Auch das Bromierungsmittel wird vorzugsweise im Ueberschuss eingesetzt.

Die Reduktion nach Verfahrensvariante d) kann beispielsweise unter Verwendung von elementarem Wasserstoff und einem geeigneten Katalysator durchgeführt werden. Diese partielle Alkin-Hydrierung, d. h. die Hydrierung des Alkins zum Alken durch Aufnahme eines Moläquivalents Wasserstoff, wird in an sich bekannter Weise durch die passende Wahl der Hydrierbedingungen bewerkstelligt. Ein dabei besonders geeigneter Katalysator ist Palladium, vorzugsweise mit Blei, Silber, Magnesium, Rhodium oder Quecksilber usw. vergiftet. Auf diese Weise werden allfällige andere reduzierbare Reste im Molekül, z. B. $C_{3-6}$-Alkenyl oder $C_{3-6}$-Alkenylthio ($R^1$), praktisch nicht hydriert, also konkurrierende Reduktionen finden kaum statt. Von den vergifteten Palladium-Katalysatoren ist der Lindlar-Katalysator [mit Blei desaktivierter Palladium-Katalysator; siehe H. Lindlar, Helv. 35, 446 (1952)] besonders bevorzugt.

Der Katalysator befindet sich zweckmässigerweise auf einem Träger, wie Calcium-, Strontium- oder Bariumcarbonat, Bariumsulfat, Kohle, Silicagel oder Aluminiumoxid. Zudem wird zweckmässigerweise in Gegenwart eines Verdünnungsmittels gearbeitet. Es kommen als Verdünnungsmittel beispielsweise niedere Alkohole, wie Methanol und Aethanol, aliphatische und cyclische Aether, wie Diäthyläther und Tetrahydrofuran, Essigsäure und deren Niederalkylester, z. B. Aethylacetat, Dimethylformamid und Pyridin in Frage. Die Hydrierung erfolgt insbesondere bei Normaldruck in einem Temperaturbereich von 0-300 °C, in vielen Fällen vorzugsweise bei Raumtemperatur.

Ausser Palladium können bei der Hydrierung aber auch andere Metallkatalysatoren verwendet werden, wie beispielsweise Nickel, insbesondere Raney-Nickel, das durch mindestens 6-monatige Lagerung unter Aethanol partiell desaktiviert ist, Raney-Eisen und Raney-Zink.

Ferner kann die Reduktion nach Verfahrensvariante d) unter Verwendung eines komplexen Hydrids, vorzugsweise Diboran (aus Natriumborhydrid und Borfluorid in situ herstellbar) oder Organo-Borhydriden, erfolgen. Durch eine solche Hydroborierung wird das Aethinderivat der Formel I''' in das entsprechende Alken-(1)-yl-boran übergeführt, das anschliessend durch Einwirkung einer Carbonsäure, vorzugsweise Essigsäure, einer Protolyse in das entsprechende cis-Alken unterworfen wird. Beispiele von Organo-borhydriden sind Bis-[3-methyl-butyl-(2)]-boran und 2,3-Dimethylbutyl-(2)-boran (aus Diboran und 2-Methyl-buten-(2) bzw. 2,3-Dimethyl-buten-(2) herstellbar). Die Umsetzung wird zweckmässigerweise in Gegenwart eines Lösungsmittels, insbesondere eines aliphatischen oder cyclischen Aethers, wie Diäthyläther oder Bis-(2-methoxyäthyl)-äther (Diglyme) in einem Temperaturbereich zwischen —80 °C und der Rückflusstemperatur des Reaktionsgemisches, vorzugsweise zwischen 0 °C und der Raumtemperatur, durchgeführt. Bei dieser an sich bekannten Methode können stereochemisch einheitliche Alkene (cis-Alkene) von hoher Reinheit hergestellt werden.

Die Reduktion nach Verfahrensvariante d) kann schliesslich auch durch partielle Elektrolyse erfolgen. Es handelt sich dabei um eine an sich bekannte, partielle elektrolytische Reduktion an Kathodenmaterialien mit geringer Ueberspannung für Wasserstoff, wie Platin, Nickel, Cobalt, Silber usw. Man arbeitet in wässrig-alkoholischen Medium in saurer oder alkalischer Lösung, in einem Temperaturbereich von 0-80 °C und bei Stromdichten von ca. 1-2 Amp/$dm^2$, wobei eine Potential-Kontrolle zu empfehlen ist.

Die N-Oxydation nach Verfahrensvariante e) wird zweckmässigerweise unter denjenigen Reaktionsbedingungen, die in der europäischen Patentpublikation Nr. 74018, Seite 10, Zeilen 10-36, für eine analoge N-Oxydation beschrieben sind, durchgeführt.

Zur Herstellung der Säureadditionssalze der Verbindungen der Formel I werden die Verbindungen I mit den gewünschten Säuren auf übliche Weise umgesetzt.

Die Isolierung und Reinigung der so hergestellten Verbindungen der Formel I bzw. der Säureadditionssalze können nach an sich bekannten Methoden erfolgen.

Die Ausgangsmaterialien der allgemeinen Formel II sind grösstenteils neu.

Diejenigen Ketone der Formel II, in der $R^1$ gegebenenfalls substituiertes $C_{1-6}$-Alkyl, $C_{3-6}$-Alkenyl oder $C_{3-6}$-Alkinyl, wie oben näher definiert, bedeutet, können hergestellt werden, indem man ein Keton der allgemeinen Formel

$$R-CH_2-\underset{\underset{O}{\|}}{C}-R^{4'} \qquad (IV)$$

worin

R und $R^{4'}$ die oben angegebenen Bedeutungen besitzen,

mit einer Verbindung der allgemeinen Formel

$$R^{1'}-Y \qquad (V)$$

worin

$R^{1'}$ gegebenenfalls mit einer $C_{1-4}$-Alkoxygruppe substituiertes $C_{1-6}$-Alkyl, $C_{3-6}$-Alkenyl oder $C_{3-6}$-Alkinyl und

Y eine Abgangsgruppe, insbesondere Chlor, Brom oder Jod, bedeuten, behandelt.

Bei der Umsetzung wird mit Vorteil das Keton der Formel IV mit einer Base, wie Natriumhydrid oder Lithium-diisopropylamid, in einem Verdünnungsmittel, vorzugsweise einem aprotischen organischen Lösungsmittel, wie Tetrahydrofuran, Dimethoxyäthan oder Dimethylformamid, bei Reaktionstemperaturen zwischen —70 °C und 50 °C in ein Anion übergeführt, welches dann mit der Verbindung der Formel V versetzt wird. Die Behandlung des Ketons der Formel IV mit der Verbindung der Formel V kann auch in einem wässrig-organischen Zweiphasensystem in Gegenwart eines Phasentransferkatalysators, d. h. unter den Bedingungen der Phasentransferkatalyse (siehe z. B. J. Dockx, Synthesis (1973), 441), erfolgen.

Diejenigen Ketone der Formel II, in der $R^1$ gegebenenfalls substituiertes $C_{1-6}$-Alkylthio, $C_{3-6}$-Alkenylthio, $C_{3-6}$-Alkinylthio oder Arylthio, wie oben näher definiert, bedeutet, können dadurch hergestellt werden, dass man einen Thioäther der allgemeinen Formel

$$R-CH_2-R^{1''} \qquad (VI)$$

worin

R die oben angegebene Bedeutung besitzt und

$R^{1''}$ gegebenenfalls mit einer $C_{1-4}$-Alkoxygruppe substituiertes $C_{1-6}$-Alkylthio, $C_{3-6}$-Alkenylthio oder $C_{3-6}$-Alkinylthio ; oder gegebenenfalls mit 1 bis 3 Halogenatomen und/oder 1 oder 2 $C_{1-3}$-Alkylgruppen und/oder 1 oder 2 $C_{1-3}$-Alkoxygruppen und/oder einer Nitrogruppe mono-, di- oder trisubstituiertes Arylthiobedeutet mit einem Ester der allgemeinen Formel

$$R^{4'}-\underset{\underset{O}{\|}}{C}-OZ \qquad (VII)$$

worin

$R^{4'}$ die oben angegebene Bedeutung besitzt und

Z $C_{1-4}$-Alkyl oder Phenyl bedeutet, umsetzt. Der Thioäther der Formel VI wird zweckmässigerweise zuerst mit einer starken Base, wie einem Alkalimetallamid, z. B. Lithiumdiisopropylamid, in einem inerten Verdünnungsmittel, wie einem Kohlenwasserstoff oder einem aliphatischen oder cyclischen Aether, z. B. Tetrahydrofuran, und bei einer Temperatur von ca. —20 °C behandelt. Der Zusatz eines Komplexierungsmittels, z. B. Hexamethylphosphorsäuretriamid, zum Reaktionsgemisch erweist sich als vorteilhaft. Anschliessend wird der Ester der Formel VII zugefügt und das Reaktionsgemisch auf Raumtemperatur gebracht. Auf diese Weise wird die Reaktion normalerweise innert kurzer Zeit beendet.

Die Ausgangsmaterialien der allgemeinen Formel III sind entweder bekannt oder können nach an sich bekannten Methoden hergestellt werden.

Bei den Ausgangsmaterialien der allgemeinen Formeln I', I'', I''' und I'''' handelt es sich um Untergruppen der Verbindungen der Formel I. Die Ausgangsmaterialien der Formeln I' und I'' sowie diejenigen Ausgangsmaterialien der Formel I''', in der $R^2$ Hydroxy bedeutet, können gemäss der Verfahrensvariante a) aus entsprechenden Ausgangsmaterialien der Formeln II und III hergestellt werden während die Ausgangsmaterialien der Formel I''', in der $R^2$ Chlor oder Brom bedeutet, gemäss der Verfahrensvariante c) aus entsprechenden Alkoholen der Formel I'' und einem Chlorierungsmittel bzw. Bromierungsmittel hergestellt werden können. Die Ausgangsmaterialien der Formel I'''' sind zugleich die Endprodukte der Verfahrensvarianten a), b), c) und d).

Die Ausgangsmaterialien der allgemeinen Formeln IV, V, VI und VII sind entweder bekannt oder können nach an sich bekannten Methoden hergestellt werden.

Die erfindungsgemässen Verbindungen, d. h. die Verbindungen der Formel I und deren Säureadditionssalze, besitzen fungizide Wirkung und können dementsprechend zur Bekämpfung von Fungi in der Landwirtschaft und im Gartenbau Verwendung finden. Sie eignen sich insbesondere zur Hemmung des Wachstums oder zur Vernichtung von phytopathogenen Pilzen auf Pflanzenteilen, z. B. Blättern, Stengeln, Wurzeln, Knollen, Früchten oder Blüten, und auf Saatgut sowie im Erdboden und sind

besonders wirksam bei der Bekämpfung von Botrytis cinerea (Graufäule) ; von echten Mehltaupilzen, wie beispielsweise Uncinula necator (Rebenmehltau), Erysiphe cichoracearum (Gurkenmehltau), Podosphaera leucotricha (Apfelmehltau) und Erysiphe graminis (Gerstenmehltau) ; von Venturia inaequalis (Apfelschorf) ; und von Schadpilzen der Gattungen Puccinia, Uromyces, Hemileia, Rhizoctonia, Penicillium, Septoria, Corticium, Cercospora, Helminthosporium und Alternaria.

Einzelne Vertreter der erfindungsgemässen Verbindungen besitzen zudem eine ausgeprägte Wirkung gegen holzzerstörende Pilze, wie z. B. Coniophora puteana und Gloeophyllum trabeum.

Die erfindungsgemässen Verbindungen zeichnen sich durch lokale und bzw. oder systemische Wirkung aus.

Die erfindungsgemässen Verbindungen wirken unter Gewäschshausdingungen bereits bei einer Konzentration von 1 mg bis 500 mg Wirksubstanz pro Liter Spritzbrühe. Im Freiland werden vorteilhaft Konzentrationen von 25 g bis 1 000 g Wirkstoff der Formel I pro Hektar und Behandlung zur Anwendung gebracht. Zur Bekämpfung von samenbürtigen Pilzen im Beizverfahren werden mit Vorteil 0,025 g bis 1,5 g Wirkstoff der Formel I pro kg Samen verwendet.

Die erfindungsgemässen Verbindungen können zu verschiedenartigen Mitteln, z. B. Lösungen, Suspensionen, Emulsionen, emulgierbaren Konzentraten und pulverförmigen Präparaten, formuliert werden. Die erfindungsgemässen fungiziden Mittel sind dadurch gekennzeichnet, dass sie eine wirksame Menge mindestens einer Verbindung der allgemeinen Formel I, wie oben definiert, oder eines Säureadditionssalzes einer solchen Verbindung sowie Formulierungshilfsstoffe enthalten. Die Mittel enthalten zweckmässigerweise zumindest einen der folgenden Formulierungshilfsstoffe :

Feste Trägerstoffe ; Lösungs- bzw. Dispersionsmittel ; Tenside (Netz- und Emulgiermittel) ; Dispergatoren (ohne Tensidwirkung) ; und Stabilisatoren.

Als feste Trägerstoffe kommen im wesentlichen in Frage : natürliche Mineralstoffe, wie Kaolin, Tonerden, Kieselgur, Talkum, Bentonit, Kreide, z. B. Schlämmkreide, Magnesiumcarbonat, Kalkstein, Quarz, Dolomit, Attapulgit, Montmorillonit und Diatomeenerde ; synthetische Mineralstoffe, wie hochdisperse Kieselsäure, Aluminiumoxid und Silikate ; organische Stoffe, wie Cellulose, Stärke, Harnstoff und Kunstharze ; und Düngemittel, wie Phosphate und Nitrate, wobei solche Trägerstoffe z. B. als Granulate oder Pulver vorliegen können.

Als Lösungs- bzw. Dispersionsmittel kommen im wesentlichen in Frage : Aromaten, wie Toluol, Xylole, Benzol und Alkylnaphthaline ; chlorierte Aromaten und chlorierte aliphatische Kohlenwasserstoffe, wie Chlorbenzole, Chloräthylene und Methylenchlorid ; aliphatische Kohlenwasserstoffe, wie Cyclohexan und Paraffine, z. B. Erdölfraktionen ; Alkohole, wie Butanol und Glykol, sowie deren Aether und Ester ; Ketone, wie Aceton, Methyläthylketon, Methylisobutylketon und Cyclohexanon ; und stark polare Lösungs- bzw. Dispersionsmittel, wie Dimethylformamid, N-Methylpyrrolidon und Dimethylsulfoxid, wobei solche Lösungs- bzw. Dispersionsmittel vorzugsweise Flammpunkte von mindestens 30 °C und Siedepunkte von mindestens 50 °C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z. B. Dichlordifluormethan. Im Falle der Benutzung von Wasser als Lösungsmittel können z. B. auch organische Lösungsmittel als Hilfslösungsmittel verwendet werden.

Die Tenside (Netz- und Emulgiermittel) können nichtionische Verbindungen sein, wie Kondensationsprodukte von Fettsäuren, Fettalkoholen oder fettsubstituierten Phenolen mit Aethylenoxid ; Fettsäureester und -äther von Zuckern oder mehrwertigen Alkoholen ; die Produkte, die aus Zuckern oder mehrwertigen Alkoholen durch Kondensation mit Aethylenoxid erhalten werden ; Blockpolymere von Aethylenoxid und Propylenoxid ; oder Alkyldimethylaminoxide.

Die Tenside können auch anionische Verbindungen darstellen, wie Seifen ; Fettsulfatester, z. B. Dodecylnatriumsulfat, Octadecylnatriumsulfat und Cetylnatriumsulfat ; Alkylsulfonate, Arylsulfonate und fettaromatische Sulfonate, wie Alkylbenzolsulfonate, z. B. Calcium-dodecylbenzolsulfonat, und Butylnaphthalinsulfonate ; und komplexere Fettsulfonate, z. B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage : Lignin, Natrium- und Ammoniumsalze von Ligninsulfonsäure, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z. B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z. B. Epichlorhydrin, Phenylglycidäther und Soyaepoxide ; Antioxidantien, z. B. Gallussäurester und Butylhydroxytoluol ; UV-Absorber, z. B. substituierte Benzophenone, Diphenylacrylnitrilsäureester und Zimtsäureester ; und Deaktivatoren, z. B. Salze der Aethylendiamintetraessigsäure und Polyglykole.

Die erfindungsgemässen fungiziden Mittel können neben den Wirkstoffen der Formel I auch andere Wirkstoffe enthalten, z. B. anderweitige fungizide Mittel, insektizide und akarizide Mittel, Bakterizide, Pflanzenwachstumsregulatoren und Düngemittel. Solche Kombinationsmittel eignen sich zur Verbreiterung des Wirkungsspektrums oder zur spezifischen Beeinflussung des Pflanzenwachstums.

Im allgemeinen enthalten die erfindungsgemässen fungiziden Mittel, je nach deren Art, zwischen 0,0 001 und 95 Gewichtsprozent an erfindungsgemässer Verbindung bzw. erfindungsgemässen Verbindungen als Wirkstoff(en). Sie können in einer Form vorliegen, die sich für die Lagerung und den Transport eignet. In solchen Formen, z. B. emulgierbaren Konzentraten, ist die Wirkstoffkonzentration normalerweise im höheren Bereich der obigen Konzentrationsreihe. Diese Formen können dann mit gleichen oder verschiedenen Formulierungshilfsstoffen bis zu Wirkstoffkonzentrationen verdünnt werden, die sich für den praktischen Gebrauch eignen, und solche Konzentrationen liegen normalerweise im niedrigeren Bereich der obigen Konzentrationsreihe. Emulgierbare Konzentrate enthalten im allgemeinen 5 bis 95 Gewichtsprozent, vorzugsweise 25 bis 75 Gewichtsprozent, der Verbindung bzw. Verbindungen der Formel I. Als Anwendungsformen kommen u. a. gebrauchsfertige Lösungen, Emulsionen und Suspensionen, die sich beispielsweise als Spritzbrühen eignen, in Frage. In solchen Spritzbrühen können z. B. Konzentrationen zwischen 0,0 001 und 20 Gewichtsprozent vorliegen. Im Ultra-Low-Volume-Verfahren können Spritzbrühen formuliert werden, in denen die Wirkstoffkonzentration vorzugsweise von 0,5 bis 20 Gewichtsprozent beträgt, während die im Low-Volume-Verfahren und im High-Volume-Verfahren formulierten Spritzbrühen vorzugsweise eine Wirkstoffkonzentration von 0,02 bis 1,0 bzw. 0,002 bis 0,1 Gewichtsprozent aufweisen.

Die erfindungsgemässen fungiziden Mittel können dadurch hergestellt werden, dass man mindestens eine Verbindung der allgemeinen Formel I bzw. ein Säureadditionssalz einer solchen Verbindung mit Formulierungshilfsstoffen vermischt.

Die Herstellung der Mittel kann in bekannter Weise durchgeführt werden, z. B. durch Vermischen der Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln oder von Dispergatoren, durch Verdünnen bereits vorbereiteter emulgierbarer Konzentrate mit Lösungs- bzw. Dispersionsmitteln, usw.

Im Falle von pulverförmigen Mitteln kann der Wirkstoff mit einem festen Trägerstoff vermischt werden, z. B. durch Zusammenmahlen ; oder man kann den festen Trägerstoff mit einer Lösung oder Suspension des Wirkstoffs imprägnieren und dann das Lösungs- bzw. Dispersionsmittel durch Abdunsten, Erhitzen oder durch Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z. B. als Spritzmittel eignen, übergeführt werden können.

Die erfindungsgemässen Verbindungen können auch mit einem Tensid und einem festen Trägerstoff zur Bildung eines netzbaren Pulvers vermischt werden, welches in Wasser dispergierbar ist, oder sie können mit einem festen vorgranulierten Trägerstoff zur Bildung eines granulatförmigen Produktes vermischt werden.

Wenn gewünscht, kann eine erfindungsgemässe Verbindung in einem mit Wasser nicht mischbaren Lösungsmittel, wie beispielsweise einem alicylischen Keton, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Mittel kann nach den im Pflanzenschutz bzw. in der Landwirtschaft üblichen Applikationsmethoden erfolgen. Das erfindungsgemässe Verfahren zur Bekämpfung von Fungi ist dadurch gekennzeichnet, dass man das zu schützende Gut, z. B. Pflanzen, Pflanzenteile bzw. Samen, mit einer wirksamen Menge einer erfindungsgemässen Verbindung bzw. eines erfindungsgemässen Mittels behandelt.

Die nachstehenden Beispiele illustrieren die Erfindung.

I. Herstellung der Wirkstoffe der Formel I :

Beispiel 1

Zu einer Lösung von 2 g Acetylen in 140 ml Tetrahydrofuran werden bei — 75 °C bis — 70 °C 48 ml n-Butyllithium (1,6 molar in n-Hexan) getropft. Nach 10-minütiger Reaktion bei — 75 °C wird eine Lösung von 10,7 g 2-(2,4-Dichlorphenyl)-1-(3-pyridyl)-1-propanon in 20 ml Tetrahydrofuran bei — 75 °C bis — 70 °C zugetropft. Nach weiteren 100 Minuten bei — 75 °C wird die Kühlung entfernt und das Reaktionsgemisch dadurch langsam auf Raumtemperatur erwärmt. Die Reaktion wird durch Zugabe von 30 ml Wasser gestoppt, und die dabei entstehende wässrige Phase wird durch Eintragen von pulverisiertem Kaliumcarbonat zu einer Paste eingedickt. Die organische Phase wird abdekantiert und die anorganische Paste dreimal mit je 25 ml Diäthyläther gewaschen. Nach Entfernen des Lösungsmittels

unter vermindertem Druck aus den organischen Phasen liegt ein braunes, hochviskoses Oel vor. Durch Zugabe von Essigsäureäthylester und n-Hexan erhält man daraus kristallines α-Aethinyl-α-(2,4-dichlor-α-methylbenzyl)-3-pyridylmethanol, Smp. 143 °C (Zersetzung).

Auf analoge Weise erhält man :

aus 2-(2,4-Dichlorphenyl)-1-(3-pyridyl)-4-penten-1-on und Acetylen das α-Aethinyl-α-(α-allyl-2,4-dichlor-benzyl)-3-pyridylmethanol, Smp. 122-123 °C ;

aus 2-(2,4-Dichlorphenyl)-1-(3-pyridyl)-1-butanon und Acetylen das α-Aethinyl-α-(α-äthyl-2,4-dichlorbenzyl)-3-pyridylmethanol, Smp. 124-124,5 °C ;

aus 2-(2,4-Dichlorphenyl)-1-(3-pyridyl)-4-pentin-1-on und Acetylen das α-Aethinyl-α-[2,4-dichlor-α-(2-propinyl) benzyl]-3-pyridylmethanol, Smp. 137-137,5 °C ;

aus 2-(2,4-Dichlorphenyl)-2-(methylthio)-1-(3-pyridyl)-1-äthanon und Acetylen das α-Aethinyl-α-(2,4-dichlor-α-methylthio-benzyl)-3-pyridylmethanol, Smp. 136-137 °C ;

aus 2-(2,4-Dichlorphenyl)-1-(3-pyridyl)-1-propanon und 1-Pentin das α-(2,4-Dichlor-α-methylbenzyl)-α-(1-pentinyl)-3-pyridylmethanol, Smp. 117-117,5 °C ;

aus 2-(2,4-Dichlorphenyl)-1-(3-pyridyl)-1-propanon und 1-Decin das α-(1-Decinyl)-α-(2,4-dichlor-α-methylbenzyl)-3-pyridylmethanol als gelbes Oel ;

aus 2-(2,4-Dichlorphenyl)-1-(3-pyridyl)-1-propanon und Phenylacetylen das α-(2,4-Dichlor-α-methylbenzyl)-α-phenyläthinyl-3-pyridylmethanol, Smp. 160 °C ;

aus 2-(2,4-Dichlorphenyl)-1-(3-pyridyl)-1-propanon und Aethoxyacetylen das α-Aethoxyäthinyl-α-(2,4-dichlor-α-methylbenzyl)-3-pyridylmethanol, Smp. 125 °C ;

aus 2-(2,4-Dichlorphenyl)-1-(3-pyridyl)-1-propanon und 1,1-Diäthoxypropin-2 das α-(3,3-Diäthoxy-1-propinyl)-α-(2,4-dichlor-α-methylbenzyl)-3-pyridylmethanol, Smp. 117 °C ;

aus 2-(2,4-Dichlorphenyl)-1-(3-pyridyl)-1-propanon und Propargylchlorid das α-(3-Chlor-1-propinyl)-α-(2,4-dichlor-α-methylbenzyl)-3-pyridylmethanol, Smp. 127 °C ;

aus 2-(4-Chlorphenyl)-1-(3-pyridyl)-1-propanon und Acetylen das α-Aethinyl-α-(4-Chlor-α-methylbenzyl)-3-pyridylmethanol, Smp. 113-119 °C ;

aus 2-(4-Chlorphenyl)-1-(3-pyridyl)-1-propanon und 1,1-Diäthoxypropin-2 das α-(4-Chlor-α-methylbenzyl)-α-(3,3-diäthoxy-1-propinyl)-3-pyridylmethanol als gelbes Oel ;

aus 2-(2,4-Dichlorphenyl)-1-(3-pyridyl)-1-pentanon und Acetylen das α-Aethinyl-α-[2,4-dichlor-α-(n-propyl)-benzyl]-3-pyridylmethanol, Smp. 141-143 °C ;

aus 2-(2,4-Dichlorphenyl)-1-(3-pyridyl)-1-pentanon und 1,1-Diäthoxypropin-2 das α-(3,3-Diäthoxy-1-propinyl)-α-[2,4-dichlor-α-(n-propyl)-benzyl]-3-pyridylmethanol, Smp. 124-125 °C ;

aus 2-(2,4-Dichlorphenyl)-1-(2-pyrazinyl)-4-penten-1-on und Acetylen das α-Aethinyl-α-(α-allyl-2,4-dichlorbenzyl)-2-pyrazinylmethanol, Smp. 108-109 °C.

## Beispiel 2

Zu einer aus 2,9 g Magnesiumspänen und 21 g 4-Bromfluorbenzol in 100 ml absolutem Tetrahydrofuran hergestellten Grignardlösung wird eine Lösung von 28 g 2-(2,4-Dichlorphenyl)-1-(3-pyridyl)-1-propanon in 50 ml absolutem Tetrahydrofuran unter Eiskühlung so zugetropft, dass die Reaktionstemperatur 30 °C nicht überschreitet. Das Reaktionsgemisch wird 1 Stunde bei Raumtemperatur nachgerührt. Anschliessend werden 100 ml 20 %ige wässrige Ammoniumchlorid-Lösung zugetropft, und die organische Phase wird abgetrennt, mit Wasser gewaschen, über wasserfreiem Natriumsulfat getrocknet und eingedampft. Der kristalline Rückstand wird zwecks Entfernung von organischen Verunreinigungen mit warmen Diäthyläther behandelt, filtriert und getrocknet. Es werden 32,2 g (85 % der theoretischen Ausbeute) α-(2,4-Dichlor-α-methylbenzyl)-α-(p-fluorphenyl)-3-pyridylmethanol, Smp. 187-188 °C, erhalten.

In analoger Weise erhält man ausgehend von :

— 2-(2,4-Dichlorphenyl)-1-(3-pyridyl)-1-propanon und 4-Chlorphenylmagnesiumchlorid das α-(2,4-Dichlor-α-methylphenyl)-α-(p-chlorphenyl)-3-pyridylmethanol, Smp. 175-176 °C ;

— 2-(2,4-Dichlorphenyl)-1-(3-pyridyl)-4-penten-1-on und 4-Chlorphenylmagnesiumchlorid das α-(α-Allyl-2,4-dichlorbenzyl)-α-(p-chlorphenyl)-3-pyridylmethanol, Smp. 93-95 °C ;

— 2-(2,4-Dichlorphenyl)-1-(3-pyridyl)-1-propanon und Allylmagnesiumbromid das α-Allyl-α-(2,4-dichlor-α-methylbenzyl)-3-pyridylmethanol, $N_D^{25}$ : 1,575 6 ;

— 2-(2,4-Dichlorphenyl)-1-(3-pyridyl)-1-propanon und Vinylmagnesiumbromid das α-(2,4-Dichlor-α-methylbenzyl)-α-vinyl-3-pyridylmethanol, Smp. 154-156 °C ;

— 2-(2,4-Dichlorphenyl)-1-(3-pyridyl)-4-penten-1-on und Vinylmagnesiumbromid das α-(α-Allyl-2,4-dichlorbenzyl)-α-vinyl-3-pyridylmethanol, Smp. 154-156 °C ;

— 2-(2,4-Dichlorphenyl)-2-methylthio-1-(3-pyridyl)-1-äthanon und Vinylmagnesiumbromid das α-(2,4-Dichlor-α-methylthio-benzyl)-α-vinyl-3-pyridylmethanol, Smp. 132-133,5 °C ;

— 2-(2,4-Dichlorphenyl)-1-(3-pyridyl)-1-propanon und 1-Propenylmagnesiumbromid das α-(2,4-Dichlor-α-methylbenzyl)-α-(1-propen-1-yl)-3-pyridylmethanol, Smp. 115-119 °C ;

— 2-(2,4-Dichlorphenyl)-1-(3-pyridyl)-1-propanon und 2-Propenylmagnesiumbromid das α-(2,4-Dichlor-α-methylbenzyl)-α-isopropenyl-3-pyridylmethanol, Smp. 169-171 °C.

Beispiel 3

150 mg α-Aethinyl-α-(2,4-dichlor-α-methylbenzyl)-3-pyridylmethanol werden in 1,5 ml Thionylchlorid gelöst und 30 Minuten bei Raumtemperatur gerührt. Das Reaktionsgemisch wird danach eingeengt, auf Eiswasser gegeben und mit gesättigter Natriumbicarbonat-Lösung neutralisiert. Extraktion mit Aethylacetat liefert nach Trocknen über wasserfreiem Natriumsulfat und vollständigem Eindampfen der organischen Phase das 3-[1-Chlor-1-(2,4-dichlor-α-methylbenzyl)-2-propinyl]-pyridin als gelbes Oel.

Auf analoge Weise erhält man :

— aus α-(2,4-Dichlor-α-methylbenzyl)-α-(1-pentinyl)-3-pyridylmethanol und Thionylchlorid das 3-[1-Chlor-1-(2,4-dichlor-α-methylbenzyl)-2-hexinyl]-pyridin als gelbes Oel ;

— aus α-(1-Decinyl)-α-(2,4-dichlor-α-methylbenzyl)-3-pyridylmethanol und Thionylchlorid das 3-[1-Chlor-1-(2,4-dichlor-α-methylbenzyl)-2-undecinyl]-pyridin als gelbes Oel.

Beispiel 4

Eine Lösung von 1 g α-Aethinyl-α-(2,4-dichlor-α-methylbenzyl)-3-pyridylmethanol in 10 ml Methanol wird bei 10 °C mit 1,4 ml 28 %iger wässriger Natriumhydroxid-Lösung und 0,83 g Jod versetzt. Die Reaktionslösung wird bei 15-20 °C während 4 Stunden gerührt und unter vermindertem Druck vom Lösungsmittel befreit. Der Rückstand wird in Aethylacetat aufgenommen und mit gesättigter Natriumchlorid-Lösung gewaschen. Nach Trocknen der organischen Phase über wasserfreiem Natriumsulfat entfernt man daraus im Wasserstrahlvakuum das Lösungsmittel und kristallisiert aus dem Rückstand durch Zugabe von Aethylacetat und n-Hexan das α-(2,4-Dichlor-α-methylbenzyl)-α-jodäthinyl-3-pyridylmethanol, Smp. 160 °C.

Auf analoge Weise erhält man :

— aus α-Aethinyl-α-(α-äthyl-2,4-dichlorbenzyl)-3-pyridylmethanol und Jod das α-(α-Aethyl-2,4-dichlorbenzyl)-α-jodäthinyl-3-pyridylmethanol, Smp. 173 °C ;

— aus α-Aethinyl-α-(2,4-dichlor-α-methylbenzyl)-3-pyridylmethanol und Brom das α-Bromäthinyl-α-(2,4-dichlor-α-methylbenzyl)-3-pyridylmethanol, Smp. 131-132 °C (Zersetzung) ;

— aus α-Aethinyl-α-(α-allyl-2,4-dichlorbenzyl)-2-pyrazinylmethanol und Jod das α-(α-Allyl-2,4-dichlorbenzyl)-α-jodäthinyl-2-pyrazinylmethanol, Smp. 123-125 °C.

Beispiel 5

Eine Lösung von 2,45 g α-(2,4-Dichlor-α-methylbenzyl)-α-(1-pentinyl)-3-pyridylmethanol in 100 ml Methanol wird mit 60 mg Lindlar-Katalysator (4,6 % Palladium und 4,6 % Blei(II)-hydroxid auf Calciumcarbonat) in einer Wasserstoffatmosphäre bei Normaldruck 7 Stunden bei Raumtemperatur gerührt. Nach Ablauf jeweils einer Stunde gibt man weitere 60 mg Lindlar-Katalysator, also insgesamt weitere 300 mg, zu. Danach wird vom Katalysator abfiltriert, das Lösungsmittel unter vermindertem Druck abdestilliert und das zurückbleibende, farblose Oel durch Zugabe von n-Hexan kristallisiert. Man erhält α-(2,4-Dichlor-α-methylbenzyl)-α-[(Z)-1-pentenyl]-3-pyridylmethanol als farblose Kristalle, Smp. 115-116 °C.

II. Formulierungsbeispiele :

Beispiel 6

1. Spritzpulver (für flüssige oder unter 75 °C schmelzende Wirkstoffe)

| | Gewichtsteile |
|---|---|
| Wirkstoff der Formel I | 50 |
| Hydratisierte Kieselsäure | 37 |
| Kaolin | 5 |
| Alkylphenoläthoxylat | 4 |
| Natrium-polynaphthalinsulfonat | 4 |
| | 100 |

Der flüssige oder geschmolzene Wirkstoff wird auf die Kieselsäure aufgezogen, die übrigen Komponenten zugemischt und das Ganze in einer geeigneten Mühle feingemahlen.

2. Spritzpulver (für feste, über 75 °C schmelzende Wirkstoffe)

| | Gewichtsteile |
|---|---|
| Wirkstoff der Formel I | 50 |
| Hydratisierte Kieselsäure | 5 |
| Kaolin | 42 |

| | |
|---|---|
| Natrium-laurylsulfat | 1 |
| Natrium-lignosulfonat | 2 |
| | 100 |

Die Komponenten werden miteinander vermischt, und das Ganze wird dann in einer geeigneten Mühle feingemahlen.

Beispiel 7

Emulgierbares Konzentrat (für bei 20-25 °C flüssige Wirkstoffe)

| | Gewichtsteile |
|---|---|
| Wirkstoff der Formel I | 500 |
| Ricinusöl-äthoxylat | 100 |
| Calcium-dodecylbenzolsulfonat | 25 |
| Gemisch von $C_{10}$-Alkylbenzolen | ad 1 000 Vol.-Teile |

Die Komponenten werden miteinander vermischt, bis eine klare Lösung entsteht.

**Patentansprüche** (für die Vertragsstaaten : BE, CH, DE, FR, GB, IT, LI, NL, SE)

1. Verbindungen der allgemeinen Formel

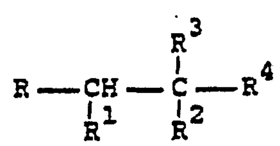

(I)

worin

R mono-, di- oder trisubstituiertes Phenyl, wobei die Substituenten 1-3 Halogenatome und/oder 1 oder 2 $C_{1-3}$-Alkylgruppen und/oder 1 oder 2 $C_{1-3}$-Alkoxygruppen und/oder 1 oder 2 Trifluormethylgruppen sind,

$R^1$ gegebenenfalls mit einer $C_{1-4}$-Alkoxygruppe substituiertes $C_{1-6}$-Alkyl, $C_{3-6}$-Alkenyl, $C_{3-6}$-Alkinyl, $C_{1-6}$-Alkylthio, $C_{3-6}$-Alkenylthio oder $C_{3-6}$-Alkinylthio ; oder gegebenenfalls mono-, di- oder trisubstituiertes Arylthio, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind,

$R^2$ Hydroxy, Chlor oder Brom,

$R^3$ $C_{2-6}$-Alkenyl ; gegebenenfalls im Arylkern mono-, di- oder trisubstituiertes Aryl oder Aryl-$C_{1-3}$-alkyl, wobei die gegebenenfalls vorhandenen Substituenten 1-3 Halogenatome und/oder 1 oder 2 $C_{1-3}$-Alkylgruppen und/oder 1 oder 2 $C_{1-3}$-Alkoxygruppen und/oder eine Nitrogruppe sind ; oder eine Gruppe $C \equiv CR^5$,

$R^4$ 3-Pyridyl, 3-Pyridyl-1-oxid, 2-Pyrazinyl, 2-Pyrazinyl-1-oxid, 2-Pyrazinyl-4-oxid, 2-Pyrazinyl-1,4-dioxid, 5-Pyrimidinyl, 5-Pyrimidinyl-1-oxid oder 5-Pyrimidinyl-1,3-dioxid und

$R^5$ Wasserstoff ; gegebenenfalls mit 1 oder 2 $C_{1-3}$-Alkoxygruppen, einem Chloratom oder einem Bromatom substituiertes $C_{1-10}$-Alkyl ; Vinyl ; $C_{1-6}$-Alkoxy ; Brom ; Jod ; oder gegebenenfalls mono-, di- oder trisubstituiertes Aryl, wobei die gegebenenfalls vorhandenen Substituenten 1-3 Halogenatome und/oder 1 oder 2 $C_{1-3}$-Alkylgruppen und/oder 1 oder 2 $C_{1-3}$-Alkoxygruppen sind, bedeuten,

und Säureadditionssalze dieser Verbindungen.

2. Verbindungen nach Anspruch 1, worin R 2,4-Dichlorphenyl bedeutet.

3. Verbindungen nach Anspruch 1 oder 2, worin $R^4$ 3-Pyridyl, 2-Pyrazinyl oder 5-Pyrimidinyl bedeutet.

4. Verbindungen nach Anspruch 1, die der Formel

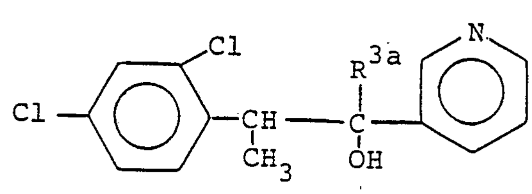

(Ia)

worin $R^{3a}$ Vinyl, 1-Propen-1-yl, Isopropenyl, Aethinyl, 1-Pentinyl, Aethoxyäthinyl oder Jodäthinyl bedeutet, entsprechen.

5. Die Verbindung nach Anspruch 1, α-Aethinyl-α-(2,4-dichlor-α-methylthio-benzyl)-3-pyridylmethanol.

6. Die Verbindung nach Anspruch 1, α-Aethinyl-α-[2,4-dichlor-α-(2-propinyl)-benzyl]-3-pyridylmethanol.

7. Die Verbindung nach Anspruch 1, α-(2,4-Dichlor-α-methylbenzyl)-α-[(Z)-1-pentenyl]-3-pyridylmethanol.

8. Verbindungen nach Anspruch 1 als fungizide Wirkstoffe.

9. Fungizides Mittel, dadurch gekennzeichnet, dass es eine wirksame Menge mindestens einer Verbindung der allgemeinen Formel I gemäss Anspruch 1, oder eines Salzes einer solchen Verbindung, sowie Formulierungshilfsstoffe enthält.

10. Fungizides Mittel nach Anspruch 9, dadurch gekennzeichnet, dass es eine wirksame Menge mindestens einer Verbindung der allgemeinen Formel Ia gemäss Anspruch 4 sowie Formulierungshilfsstoffe enthält.

11. Fungizides Mittel nach Anspruch 9, dadurch gekennzeichnet, dass es eine wirksame Menge von α-Aethinyl-α-(2,4-dichlor-α-methylthio-benzyl)-3-pyridylmethanol sowie Formulierungshilfsstoffe enthält.

12. Fungizides Mittel nach Anspruch 9, dadurch gekennzeichnet, dass es eine wirksame Menge von α-Aethinyl-α-[2,4-dichlor-α-(2-propinyl)-benzyl]-3-pyridylmethanol sowie Formulierungshilfsstoffe enthält.

13. Fungizides Mittel nach Anspruch 9, dadurch gekennzeichnet, dass es eine wirksame Menge von α-(2,4-Dichlor-α-methylbenzyl)-α-[(Z)-1-pentenyl]-3-pyridylmethanol sowie Formulierungshilfsstoffe enthält.

14. Verfahren zur Herstellung von Verbindungen der allgemeinen Formel I gemäss Anspruch 1, und von deren Säureadditionssalzen, dadurch gekennzeichnet, dass man

a) zwecks Herstellung der Verbindungen der Formel I, in der $R^2$ Hydroxy und $R^4$ 3-Pyridyl, 2-Pyrazinyl oder 5-Pyrimidinyl bedeuten und $R^3$ — mit Ausnahme von $C \equiv CBr$ oder $C \equiv CJ$ — die obige Bedeutung besitzt, ein Keton der allgemeinen Formel

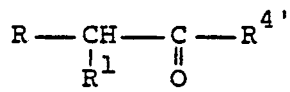

(II)

worin

$R$ und $R^1$ die oben angegebenen Bedeutungen besitzen und

$R^{4'}$ 3-Pyridyl, 2-Pyrazinyl oder 5-Pyrimidinyl bedeutet,

mit einer Verbindung der allgemeinen Formel

$$(R^{3'})_nX \qquad \text{(III)}$$

worin

$R^{3'}$ die Bedeutung von $R^3$, mit Ausnahme von $C \equiv CBr$ oder $C \equiv CJ$, besitzt und

X ein Alkalimetall, ein Erdalkalimetall oder MgHal,

n die Wertigkeit von X und

Hal Halogen bedeuten, umsetzt,

b) zwecks Herstellung der Verbindungen der Formel I, in der $R^2$ Hydroxy, $R^3$ die Gruppe $C \equiv CBr$ oder $C \equiv CJ$ und $R^4$ 3-Pyridyl, 2-Pyrazinyl oder 5-Pyrimidinyl bedeuten, ein Aethinderivat der allgemeinen Formel

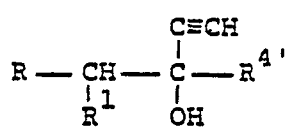

(I')

worin $R$, $R^1$ und $R^{4'}$ die oben angegebenen Bedeutungen besitzen, mit einem Bromierungsmittel bzw. Jodierungsmittel behandelt,

c) zwecks Herstellung der Verbindungen der Formel I, worin $R^2$ Chlor oder Brom und $R^4$ 3-Pyridyl, 2-Pyrazinyl oder 5-Pyrimidinyl bedeuten, einen Alkohol der allgemeinen Formel

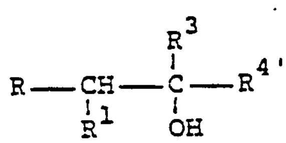

(I'')

worin R, $R^1$, $R^3$ und $R^{4'}$ die oben angegebenen Bedeutungen besitzen, mit einem Chlorierungsmittel bzw. Bromierungsmittel behandelt,

d) zwecks Herstellung der Verbindungen der Formel I, worin $R^3$ A-$C_{2-6}$-Alkenyl und $R^4$ 3-Pyridyl, 2-Pyrazinyl oder 5-Pyrimidinyl bedeuten, ein Alkinderivat der allgemeinen Formel

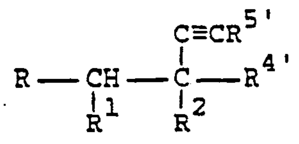

(I''')

worin

R, $R^1$, $R^2$ und $R^{4'}$ die oben angegebenen Bedeutungen besitzen und

$R^{5'}$ Wasserstoff oder $C_{1-4}$-Alkyl bedeutet, reduziert, oder

e) zwecks Herstellung der Verbindungen der Formel I, worin $R^4$ 3-Pyridyl-1-oxid, 2-Pyrazinyl-1-oxid, 2-Pyrazinyl-4-oxid, 2-Pyrazinyl-1,4-dioxid, 5-Pyrimidinyl-1-oxid oder 5-Pyrimidinyl-1,3-dioxid bedeutet, ein Pyridin-, Pyrazin- bzw. Pyrimidinderivat der allgemeinen Formel

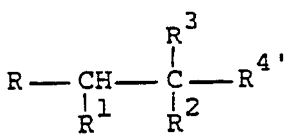

(I'''')

worin R, $R^1$, $R^2$, $R^3$ und $R^{4'}$ die oben angegebenen Bedeutungen besitzen, N-oxydiert und erwünschtenfalls eine erhaltene Verbindung der Formel I durch Umsetzung mit einer Säure in das entsprechende Säureadditionssalz überführt.

15. Verfahren zur Bekämpfung von Fungi in der Landwirtschaft und im Gartenbau, dadurch gekennzeichnet, dass man das zu schützende Gut mit einer wirksamen Menge einer Verbindung gemäss einem der Ansprüche 1 bis 6 bzw. eines Mittels gemäss einem der Ansprüche 9 bis 12 behandelt.

16. Verfahren zur Bekämpfung von Fungi in der Landwirtschaft und im Gartenbau, dadurch gekennzeichnet, dass man das zu schützende Gut mit einer wirksamen Menge einer Verbindung gemäss Anspruch 7 bzw. eines Mittels gemäss Anspruch 13 behandelt.

17. Verwendung einer Verbindung gemäss einem der Ansprüche 1 bis 6 bzw. eines Mittels gemäss einem der Ansprüche 9 bis 12 zur Bekämpfung von Fungi in der Landwirtschaft und im Gartenbau.

18. Verwendung einer Verbindung gemäss Anspruch 7 bzw. eines Mittels gemäss Anspruch 13 zur Bekämpfung von Fungi in der Landwirtschaft und im Gartenbau.

**Patentansprüche** (für den Vertragsstaat AT)

1. Fungizides Mittel, dadurch gekennzeichnet, dass es eine wirksame Menge mindestens einer Verbindung der allgemeinen Formel

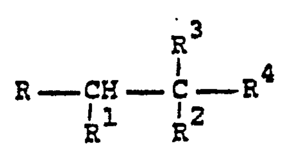

(I)

worin

R mono-, di- oder trisubstituiertes Phenyl, wobei die Substituenten 1-3 Halogenatome und/oder 1 oder 2 $C_{1-3}$-Alkylgruppen und/oder 1 oder 2 $C_{1-3}$-Alkoxygruppen und/oder 1 oder 2 Trifluormethylgruppen sind,

$R^1$ gegebenenfalls mit einer $C_{1-4}$-Alkoxygruppe substituiertes $C_{1-6}$-Alkyl, $C_{3-6}$-Alkenyl, $C_{3-6}$-Alkinyl, $C_{1-6}$-Alkylthio, $C_{3-6}$-Alkenylthio oder $C_{3-6}$-Alkinylthio ; oder gegebenenfalls mono-, di- oder trisubstituiertes Arylthio, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind,

$R^2$ Hydroxy, Chlor oder Brom,

$R^3$ $C_{2-6}$-Alkenyl ; gegebenenfalls im Arylkern mono-, di- oder trisubstituiertes Aryl oder Aryl-$C_{1-3}$-alkyl, wobei die gegebenenfalls vorhandenen Substituenten 1-3 Halogenatome und/oder 1 oder 2 $C_{1-3}$-Alkylgruppen und/oder 1 oder 2 $C_{1-3}$-Alkoxygruppen und/oder eine Nitrogruppe sind ; oder eine Gruppe $C \equiv CR^5$,

$R^4$ 3-Pyridyl, 3-Pyridyl-1-oxid, 2-Pyrazinyl, 2-Pyrazinyl-1-oxid, 2-Pyrazinyl-4-oxid, 2-Pyrazinyl-1,4-dioxid, 5-Pyrimidinyl, 5-Pyrimidinyl-1-oxid oder 5-Pyrimidinyl-1,3-dioxid

und

$R^5$ Wasserstoff ; gegebenenfalls mit 1 oder 2 $C_{1-3}$-Alkoxygruppen, einem Chloratom oder einem Bromatom substituiertes $C_{1-10}$-Alkyl ; Vinyl ; $C_{1-6}$-Alkoxy ; Brom ; Jod ; oder gegebenenfalls mono-, di- oder trisubstituiertes Aryl, wobei die gegebenenfalls vorhandenen Substituenten 1-3 Halogenatome und/oder 1 oder 2 $C_{1-3}$-Alkylgruppen und/oder 1 oder 2 $C_{1-3}$-Alkoxygruppen sind, bedeuten, oder eines Säureadditionssalzes einer solchen Verbindung, sowie Formulierungshilfsstoffe enthält.

2. Fungizides Mittel nach Anspruch 1, worin R 2,4-Dichlorphenyl bedeutet.

3. Fungizides Mittel nach Anspruch 1 oder 2, worin $R^4$ 3-Pyridyl, 2-Pyrazinyl oder 5-Pyrimidinyl bedeutet.

4. Fungizides Mittel nach Anspruch 1, dadurch gekennzeichnet, dass es eine wirksame Menge mindestens einer Verbindung der Formel

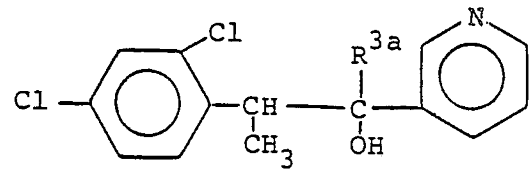

(Ia)

worin $R^{3a}$ Vinyl, 1-Propen-1-yl, Isopropenyl, Aethinyl, 1-Pentinyl, Aethoxyäthinyl oder Jodäthinyl bedeutet, sowie Formulierungshilfsstoffe enthält.

5. Fungizides Mittel nach Anspruch 1, dadurch gekennzeichnet, dass es eine wirksame Menge von α-Aethinyl-α-(2,4-dichlor-α-methylthio-benzyl)-3-pyridylmethanol sowie Formulierungshilfsstoffe enthält.

6. Fungizides Mittel nach Anspruch 1, dadurch gekennzeichnet, dass es eine wirksame Menge von α-Aethinyl-α-[2,4-dichlor-α-(2-propinyl)-benzyl]-3-pyridylmethanol sowie Formulierungshilfsstoffe enthält.

7. Fungizides Mittel nach Anspruch 1, dadurch gekennzeichnet, dass es eine wirksame Menge von α-(2,4-Dichlor-α-methylbenzyl)-α-[(Z)-1-pentenyl]-3-pyridylmethanol sowie Formulierungshilfsstoffe enthält.

8. Verfahren zur Herstellung von Verbindungen der allgemeinen Formel I gemäss Anspruch 1 und von deren Säureadditionssalzen, dadurch gekennzeichnet, dass man

a) zwecks Herstellung der Verbindungen der Formel I, in der $R^2$ Hydroxy und $R^4$ 3-Pyridyl, 2-Pyrazinyl oder 5-Pyrimidinyl bedeuten und $R^3$ — mit Ausnahme von $C \equiv CBr$ oder $C \equiv CJ$ — die obige Bedeutung besitzt, ein Keton der allgemeinen Formel

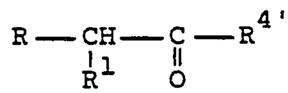

(II)

worin

R und $R^1$ die oben angegebenen Bedeutungen besitzen und

$R^{4'}$ 3-Pyridyl, 2-Pyrazinyl oder 5-Pyrimidinyl bedeutet,

mit einer Verbindung der allgemeinen Formel

$$(R^{3'})_nX \quad \text{(III)}$$

worin

$R^{3'}$ die Bedeutung von $R^3$, mit Ausnahme von $C \equiv CBr$ oder $C \equiv CJ$, besitzt und

X ein Alkalimetall, ein Erdalkalimetall oder MgHal,

n die Wertigkeit von X und

Hal Halogen bedeuten, umsetzt,

b) zwecks Herstellung der Verbindungen der Formel I, in der $R^2$ Hydroxy, $R^3$ die Gruppe $C \equiv CBr$ oder $C \equiv CJ$ und $R^4$ 3-Pyridyl, 2-Pyrazinyl oder 5-Pyrimidinyl bedeuten, ein Aethinderivat der allgemeinen Formel

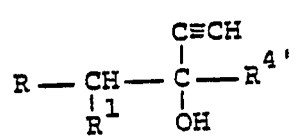

(I')

worin

R, $R^1$ und

$R^{4'}$ die oben angegebenen Bedeutungen besitzen,

mit einem Bromierungsmittel bzw. Jodierungsmittel behandelt,

c) zwecks Herstellung der Verbindungen der Formel I, worin $R^2$ Chlor oder Brom und $R^4$ 3-Pyridyl, 2-Pyrazinyl oder 5-Pyrimidinyl bedeuten, einen Alkohol der allgemeinen Formel

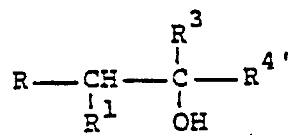

(I'')

worin

R, $R^1$, $R^3$ und $R^{4'}$ die oben angegebenen Bedeutungen besitzen,

mit einem Chlorierungsmittel bzw. Bromierungsmittel behandelt,

d) zwecks Herstellung der Verbindungen der Formel I, worin $R^3$ 1-$C_{2-6}$-Alkenyl und $R^4$ 3-Pyridyl, 2-Pyrazinyl oder 5-Pyrimidinyl bedeuten, ein Alkinderivat der allgemeinen Formel

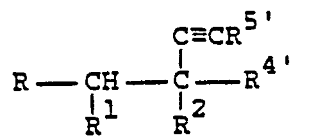

(I''')

worin

R, $R^1$, $R^2$ und $R^{4'}$ die oben angegebenen Bedeutungen besitzen und

$R^{5'}$ Wasserstoff oder $C_{1-4}$-Alkyl bedeutet, reduziert, oder

e) zwecks Herstellung der Verbindungen der Formel I, worin $R^4$ 3-Pyridyl-1-oxid, 2-Pyrazinyl-1-oxid, 2-Pyrazinyl-4-oxid, 2-Pyrazinyl-1,4-dioxid, 5-Pyrimidinyl-1-oxid oder 5-Pyrimidinyl-1,3-dioxid bedeutet, ein Pyridin-, Pyrazin- bzw. Pyrimidinderivat der allgemeinen Formel

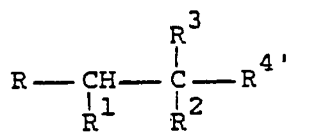

(I'''')

worin

R, $R^1$, $R^2$, $R^3$ und $R^{4'}$ die oben angegebenen Bedeutungen besitzen, N-oxydiert

und erwünschtenfalls eine erhaltene Verbindung der Formel I durch Umsetzung mit einer Säure in das entsprechende Säureadditionssalz überführt.

9. Verfahren zur Herstellung eines fungiziden Mittels, dadurch gekennzeichnet, dass man mindestens eine der in Anspruch 1 genannten Verbindungen mit Formulierungshilfsstoffen vermischt.

10. Verfahren zur Bekämpfung von Fungi in der Landwirtschaft und im Gartenbau, dadurch gekennzeichnet, dass man das zu schützende Gut mit einer wirksamen Menge einer Verbindung bzw. eines Mittels gemäss einem der Ansprüche 1 bis 6 behandelt.

11. Verfahren zur Bekämpfung von Fungi in der Landwirtschaft und im Gartenbau, dadurch gekennzeichnet, dass man das zu schützende Gut mit einer wirksamen Menge einer Verbindung bzw. eines Mittels gemäss Anspruch 7 behandelt.

12. Verwendung einer Verbindung bzw. eines Mittels gemäss einem der Ansprüche 1 bis 6 zur Bekämpfung von Fungi in der Landwirtschaft und im Gartenbau.

13. Verwendung einer Verbindung bzw. eines Mittels gemäss Anspruch 7 zur Bekämpfung von Fungi in der Landwirtschaft und im Gartenbau.

**Claims** (for the Contracting States : BE, CH, DE, FR, GB, IT, LI, NL, SE)

1. Compounds of the general formula

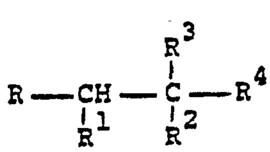

(I)

wherein

R signifies mono-, di- or trisubstituted phenyl, the substituents being 1-3 halogen atoms and/or 1 or 2

$C_{1-3}$-alkyl groups and/or 1 or 2 $C_{1-3}$-alkoxy groups and/or 1 or 2 trifluoromethyl groups,

$R^1$ signifies $C_{1-6}$-alkyl, $C_{3-6}$-alkenyl, $C_{3-6}$-alkynyl, $C_{1-6}$alkylthio, $C_{3-6}$-alkenylthio or $C_{3-6}$-alkynylthio optionally substituted with a $C_{1-4}$-alkoxy group ; or optionally mono-, di- or trisubstituted arylthio, the optionally present substituents being 1 to 3 halogen atoms ans/or 1 or 2 $C_{1-3}$-alkyl groups and/or 1 or 2 $C_{1-3}$-alkoxy groups and/or a nitro group,

$R^2$ signifies hydroxy, chlorine or bromine,

$R^3$ signifies $C_{2-6}$-alkenyl ; aryl or aryl-$C_{1-3}$-alkyl optionally mono-, di- or trisubstituted in the aryl nucleus, the optionally present substituents being 1-3 halogen atoms and/or 1 or 2 $C_{1-3}$-alkyl groups and/or 1 or 2 $C_{1-3}$-alkoxy groups and/or a nitro group ; or a group $C \equiv R^5$,

$R^4$ signifies 3-pyridyl, 3-pyridyl 1-oxide, 2-pyrazinyl, 2-pyrazinyl 1-oxide, 2-pyrazinyl 4-oxide, 2-pyrazinyl 1,4-dioxide, 5-pyrimidinyl, 5-pyrimidinyl 1-oxide or 5-pyrimidinyl 1,3-dioxide and

$R^5$ signifies hydrogen ; $C_{1-10}$-alkyl optionally substituted with 1 or 2 $C_{1-3}$-alkoxy groups, a chlorine atom or a bromine atom ; vinyl ; $C_{1-6}$-alkoxy ; bromine ; iodine ; or optionally mono-, di- or trisubstituted aryl, the optionally present substituents being 1-3 halogen atoms and/or 1 or 2 $C_{1-3}$-alkyl groups and/or 1 or 2 $C_{1-3}$-alkoxy groups,

and acid addition salts of these compounds.

2. Compounds according to claim 1, wherein R signifies 2,4-dichlorophenyl.

3. Compounds according to claim 1 or 2, wherein $R^4$ signifies 3-pyridyl, 2-pyrazinyl or 5-pyrimidinyl.

4. Compounds according to claim 1, which correspond to the formula

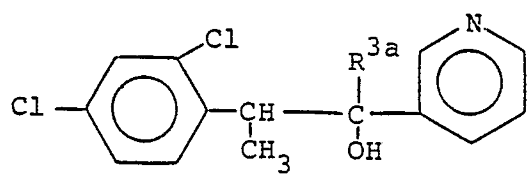

(Ia)

wherein $R^{3a}$ signifies vinyl, 1-propen-1-yl, isopropenyl, ethynyl, 1-pentynyl, ethoxyethynyl or iodoethynyl.

5. The compound according to claim 1, α-ethynyl-α-(2,4-dichloro-α-methylthio-benzyl)-3-pyridylmethanol.

6. The compound according to claim 1, α-ethynyl-α-[2,4-dichloro-α-(2-propynyl)-benzyl]-3-pyridylmethanol.

7. The compound according to claim 1, α-(2,4-dichloro-α-methylbenzyl)-α-[(Z)-1-pentenyl]-3-pyridylmethanol.

8. Compounds according to claim 1 as fungicidal active substances.

9. A fungicidal composition, characterized in that it contains an effective amount of at least one compound of general formula I in accordance with claim 1, or of a salt of such a compound, as well as formulation adjuvants.

10. A fungicidal composition according to claim 9, characterized in that it contains an effective amount of at least one compound of general formula Ia in accordance with claim 4 as well as formulation adjuvants.

11. A fungicidal composition according to claim 9, characterized in that it contains an effective amount of α-ethynyl-α-(2,4-dichloro-α-methylthio-benzyl)-3-pyridylmethanol as well as formulation adjuvants.

12. A fungicidal composition according to claim 9, characterized in that it contains an effective amount of α-ethynyl-α-[2,4-dichloro-α-(2-propynyl)-benzyl]-3-pyridylmethanol as well as formulation adjuvants.

13. A fungicidal composition according to claim 9, characterized in that it contains an effective amount of α-(2,4-dichloro-α-methylbenzyl)-α-[(Z)-1-pentenyl]-3-pyridylmethanol as well as formulation adjuvants.

14. A process for the manufacture of compounds of the general formula I in accordance with claim 1, and of their acid addition salts, characterized by

a) for the manufacture of the compounds of formula I in which $R^2$ signifies hydroxy and $R^4$ signifies 3-pyridyl, 2-pyrazinyl or 5-pyrimidinyl and $R^3$ has the above significance with the exception of $C \equiv CBr$ or $C \equiv CI$, reacting a ketone of the general formula

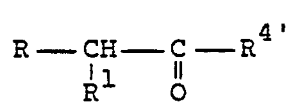

(II)

wherein

R and $R^1$ have the significances given above and

$R^{4'}$ signifies 3-pyridyl, 2-pyrazinyl or 5-pyrimidinyl,

with a compound of the general formula

$$(R^{3'})_nX \qquad (III)$$

wherein

$R^{3'}$ has the significance of $R^3$ with the exception of $C \equiv CBr$ or $C \equiv CI$, and

X signifies an alkali metal, an alkaline earth metal or MgHal,

n signifies the valency of X and

Hal signifies halogen,

b) for the manufacture of the compounds of formula I in which $R^2$ signifies hydroxy, $R^3$ signifies the group $C \equiv CBr$ or $C \equiv CI$ and $R^4$ signifies 3-pyridyl, 2-pyrazinyl or 5-pyrimidinyl, treating an ethyne derivative of the general formula

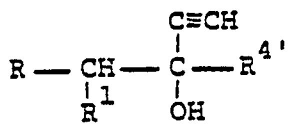

(I')

wherein

R, $R^1$ and $R^{4'}$ have the significances given above, with a brominating agent or iodinating agent,

c) for the manufacture of the compounds of formula I in which $R^2$ signifies chlorine or bromine and $R^4$ signifies 3-pyridyl, 2-pyrazinyl or 5-pyrimidinyl, treating an alcohol of the general formula

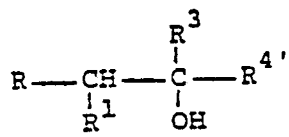

(I'')

wherein

R, $R^1$, $R^3$ and $R^{4'}$ have the significances given above, with a chlorinating agent or brominating agent,

d) for the manufacture of the compounds of formula I in which $R^3$ signifies 1-$C_{2-6}$-alkenyl and $R^4$ signifies 3-pyridyl, 2-pyrazinyl or 5-pyrimidinyl, reducing an alkyne derivative of the general formula

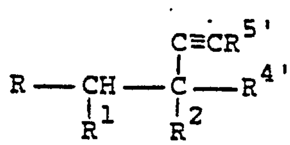

(I''')

wherein

R, $R^1$, $R^2$ and $R^{4'}$ have the significances given above and

$R^{5'}$ signifies hydrogen or $C_{1-4}$-alkyl, or

e) for the manufacture of the compounds of formula I in which $R^4$ signifies 3-pyridyl 1-oxide, 2-pyrazinyl 1-oxide, 2-pyrazinyl 4-oxide, 2-pyrazinyl 1,4-dioxide, 5-pyrimidinyl 1-oxide or 5-pyrimidinyl 1,3-dioxide, N-oxidizing a pyridine, pyrazine or pyrimidine derivative of the general formula

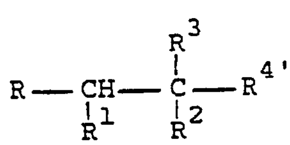

(I'''')

wherein

R, $R^1$, $R^2$, $R^3$ and $R^{4'}$ have the significances given above, and, if desired, converting a compound of formula I obtained into the corresponding acid addition salt by reaction with an acid.

15. A method for the control of fungi in agriculture and in horticulture, characterized by treating the locus to be protected with an effective amount of a compound in accordance with any one of claims 1 to 6 or of a composition in accordance with any one of claims 9 to 12.

16. A method for the control of fungi in agriculture and in horticulture, characterized by treating the locus to be protected with an effective amount of a compound in accordance with claim 7 or of a composition in accordance with claim 13.

17. The use of a compound in accordance with any one of claims 1 to 6 or of a composition in accordance with any one of claims 9 to 12 for the control of fungi in agriculture and in horticulture.

18. The use of a compound in accordance with claim 7 or of a composition in accordance with claim 13 for the control of fungi in agriculture and in horticulture.

**Claims** (for the Contracting State AT)

1. A fungicidal composition, characterized in that it contains an effective amount of at least one compound of the general formula

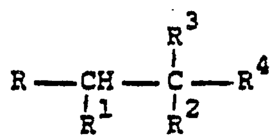

(I)

wherein

R signifies mono-, di- or trisubstituted phenyl, the substituents being 1-3 halogen atoms and/or 1 or 2 $C_{1-3}$-alkyl groups and/or 1 or 2 $C_{1-3}$-alkoxy groups and/or 1 or 2 trifluoromethyl groups,

$R^1$ signifies $C_{1-6}$-alkyl, $C_{3-6}$-alkenyl, $C_{3-6}$-alkynyl, $C_{1-6}$-alkylthio, $C_{3-6}$-alkenylthio or $C_{3-6}$-alkynylthio optionally substituted with a $C_{1-4}$-alkoxy group ; or optionally mono-, di- or trisubstituted arylthio, the optionally present substituents being 1 to 3 halogen atoms and/or 1 or 2 $C_{1-3}$-alkyl groups and/or 1 or 2 $C_{1-3}$-alkoxy groups and/or a nitro group,

$R^2$ signifies hydroxy, chlorine or bromine,

$R^3$ signifies $C_{2-6}$-alkenyl ; aryl or aryl-$C_{1-3}$-alkyl optionally mono-, di- or trisubstituted in the aryl nucleus, the optionally present substituents being 1-3 halogen atoms and/or 1 or 2 $C_{1-3}$-alkyl groups and/or 1 or 2 $C_{1-3}$-alkoxy groups and/or a nitro group ; or a group $C{\equiv}CR^5$,

$R^4$ signifies 3-pyridyl, 3-pyridyl 1-oxide, 2-pyrazinyl, 2-pyrazinyl 1-oxide, 2-pyrazinyl 4-oxide, 2-pyrazinyl 1,4-dioxide, 5-pyrimidinyl, 5-pyrimidinyl 1-oxide or 5-pyrimidinyl 1,3-dioxide and

$R^5$ signifies hydrogen ; $C_{1-10}$-alkyl optionally substituted with 1 or 2 $C_{1-3}$-alkoxy groups, a chlorine atom or a bromine atom ; vinyl ; $C_{1-6}$-alkoxy ; bromine ; iodine ; or optionally mono-, di- or trisubstituted aryl, the optionally present substituents being 1-3 halogen atoms and/or 1 or 2 $C_{1-3}$-alkyl groups and/or 1 or 2 $C_{1-3}$-alkoxy groups,

or of an acid addition salt of such a compound, as well as formulation adjuvants.

2. A fungicidal composition according to claim 1, wherein R signifies 2,4-dichlorophenyl.

3. A fungicidal composition according to claim 1 or 2, wherein $R^4$ signifies 3-pyridyl, 2-pyrazinyl or 5-pyrimidinyl.

4. A fungicidal composition according to claim 1, characterized in that it contains an effective amount of at least one compound of the formula

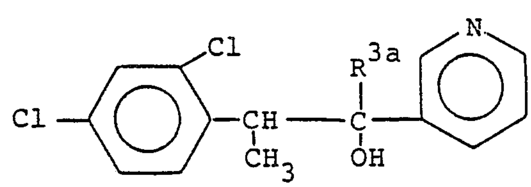

. (Ia)

wherein $R^{3a}$ signifies vinyl, 1-propen-1-yl, isopropenyl, ethynyl, 1-pentynyl, ethoxyethynyl or iodoethynyl, as well as formulation adjuvants.

5. A fungicidal composition according to claim 1, characterized in that it contains an effective amount of α-ethynyl-α-(2,4-dichloro-α-methylthio-benzyl)-3-pyridylmethanol as well as formulation adjuvants.

6. A fungicidal composition according to claim 1, characterized in that it contains an effective amount of α-ethynyl-α-[2,4-dichloro-α-(2-propynyl)-benzyl]-3-pyridylmethanol as well as formulation adjuvants.

7. A fungicidal composition according to claim 1, characterized in that it contains an effective amount of α-(2,4-dichloro-α-methylbenzyl)-α-[(Z)-1-pentenyl]-3-pyridylmethanol as well as formulation adjuvants.

8. A process for the manufacture of compounds of general formula I in accordance with claim 1 and of their acid addition salts, characterized by

a) for the manufacture of the compounds of formula I in which $R^2$ signifies hydroxy and $R^4$ signifies 3-pyridyl, 2-pyrazinyl or 5-pyrimidinyl and $R^3$ has the above significance with the exception of $C{\equiv}CBr$ or $C{\equiv}CI$, reacting a ketone of the general formula

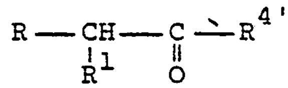

(II)

wherein

R and $R^1$ have the significances given above and

$R^{4'}$ signifies 3-pyridyl, 2-pyrazinyl or 5-pyrimidinyl,

with a compound of the general formula

$$(R^{3'})_nX \qquad (III)$$

wherein

$R^{3'}$ has the significance of $R^3$ with the exception of C≡CBr or C≡CI, and

X signifies an alkali metal, an alkaline earth metal or MgHal,

n signifies the valency of X and

Hal signifies halogen,

b) for the manufacture of the compounds of formula I in which $R^2$ signifies hydroxy, $R^3$ signifies the group C≡CBr or C≡CI and $R^4$ signifies 3-pyridyl, 2-pyrazinyl or 5-pyrimidinyl, treating an ethyne derivative of the general formula

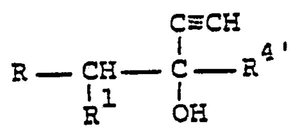

(I')

wherein

R, $R^1$ and $R^{4'}$ have the significances given above, with a brominating agent or iodinating agent,

c) for the manufacture of the compounds of formula I in which $R^2$ signifies chlorine or bromine and $R^4$ signifies 3-pyridyl, 2-pyrazinyl or 5-pyrimidinyl, treating an alcohol of the general formula

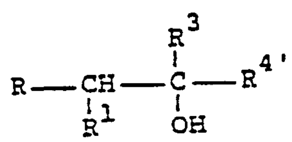

(I'')

wherein

R, $R^1$, $R^3$ and $R^{4'}$ have the significances given above, with a chlorinating agent or brominating agent,

d) for the manufacture of the compounds of formula I in which $R^3$ signifies 1-$C_{2-6}$-alkenyl and $R^4$ signifies 3-pyridyl, 2-pyrazinyl or 5-pyrimidinyl, reducing an alkyne derivative of the general formula

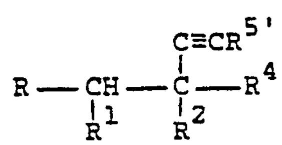

(I''')

wherein

R, $R^1$, $R^2$ and $R^{4'}$ have the significances given above and

$R^{5'}$ signifies hydrogen or $C_{1-4}$-alkyl, or

e) for the manufacture of the compounds of formula I in which $R^4$ signifies 3-pyridyl 1-oxide, 2-pyrazinyl 1-oxide, 2-pyrazinyl 4-oxide, 2-pyrazinyl 1,4-dioxide, 5-pyrimidinyl 1-oxide or 5-pyrimidinyl 1,3-dioxide, N-oxidizing a pyridine, pyrazine or pyrimidine derivative of the general formula

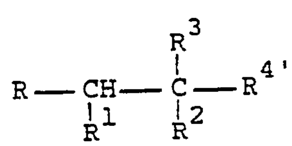

(I'''')

wherein

R, $R^1$, $R^2$, $R^3$ and $R^{4'}$ have the significances given above, and, if desired, converting a compound of formula I obtained into the corresponding acid addition salt by reaction with an acid.

9. A process for the manufacture of a fungicidal composition, characterized by mixing at least one of

the compounds set forth in claim 1 with formulation adjuvants.

10. A method for the control of fungi in agriculture and in horticulture, characterized by treating the locus to be protected with an effective amount of a compound or of a composition in accordance with any one of claims 1 to 6.

11. A method for the control of fungi in agriculture and in horticulture, characterized by treating the locus to be protected with an effective amount of a compound or of a composition in accordance with claim 7.

12. The use of a compound or of a composition in accordance with any one of claims 1 to 6 for the control of fungi in agriculture and horticulture.

13. The use of a compound or of a composition in accordance with claim 7 for the control of fungi in agriculture and horticulture.

**Revendications** (pour les Etats contractants : BE, CH, DE, FR, GB, IT, LI, NL, SE)

1. Composés de formule générale

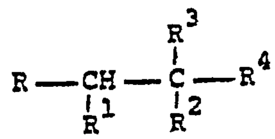

(I)

dans laquelle

R représente un groupe phényle mono-, di- ou tri-substitué, les substituants consistant en 1 à 3 atomes d'halogène et/ou 1 ou 2 groupes alkyle en C 1-C 3 et/ou 1 ou 2 groupes alcoxy en C 1-C 3 et/ou 1 ou 2 groupes trifluorométhyle,

$R^1$ représente un groupe alkyle en C 1-C 6, alcényle en C 3-C 6, alcynyle en C 3-C 6, alkylthio en C 1-C 6, alcénylthio en C 3-C 6 ou alcynylthio en C 3-C 6, éventuellement substitué par un groupe alcoxy en C 1-C 4 ; ou bien un groupe arylthio éventuellement mono-, di- ou tri-substitué, les substituants éventuels consistant en 1 à 3 atomes d'halogène et/ou un ou deux groupes alkyle en C 1-C 3 et/ou un ou deux groupes alcoxy en C 1-C 3 et/ou un groupe nitro,

$R^2$ représente un groupe hydroxy, le chlore ou le brome,

$R^3$ représente un groupe alcényle en C 2-C 6 ; un groupe aryle ou aryl-alkyle en C 1-C 3 éventuellement mono-, di- ou tri-substitué dans le noyau aryle, les substituants éventuels consistant en 1 à 3 atomes d'halogène et/ou un ou deux groupes alkyle en C 1-C 3 et/ou un ou deux groupes alcoxy en C 1-C 3 et/ou un groupe nitro ; ou un groupe $C{\equiv}CR^5$,

$R^4$ représente un groupe 3-pyridyle, 1-oxyde de 3-pyridyle, 2-pyrazinyle, 1-oxyde de 2-pyrazinyle, 4-oxyde de 2-pyrazinyle, 1,4-dioxyde de 2-pyrazinyle, 5-pyrimidinyle, 1-oxyde de 5-pyrimidinyle ou 1,3-dioxyde de 5-pyrimidinyle, et

$R^5$ représente l'hydrogène, un groupe alkyle en C 1-C 10 éventuellement substitué par un ou deux groupes alcoxy en C 1-C 3, un atome de chlore ou un atome de brome ; un groupe vinyle ; un groupe alcoxy en C 1-C 6 ; le brome ; l'iode ; ou un groupe aryle éventuellement mono-, di- ou tri-substitué, les substituants éventuels consistant en 1 à 3 atomes d'halogène et/ou un ou deux groupes alkyle en C 1-C 3 et/ou un ou deux groupes alcoxy en C 1-C 3,

et les sels de ces composés formés par addition avec des acides.

2. Composés selon la revendication 1, dans lesquels R représente le groupe 2,4-dichlorophényle.

3. Composés selon la revendication 1 ou 2, dans lesquels $R^4$ représente un groupe 3-pyridyle, 2-pyrazinyle ou 5-pyrimidinyle.

4. Composés selon la revendication 1, répondant à la formule

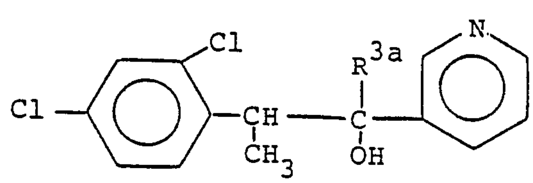

(Ia)

dans laquelle $R^{3a}$ représente un groupe vinyle, 1-propène-1-yle, isopropényle, éthynyle, 1-pentynyle, éthoxyéthynyle ou iodoéthynyle.

5. Le composé selon la revendication 1 : alpha-éthynyl-alpha-(2,4-dichloro-alpha-méthylthiobenzyl)-3-pyridylméthanol.

6. Le composé selon la revendication 1 : alpha-éthynyl-alpha-[2,4-dichloro-alpha-(2-propynyl)-benzyl]-3-pyridylméthanol.

7. Le composé selon la revendication 1 : alpha-(2,4-dichloro-alpha-méthylbenzyl)-alpha-[(Z)-1-penté-

nyl]-3-pyridylméthanol.

8. Les composés selon la revendication 1, en tant que substances actives fongicides.

9. Produit fongicide caractérisé en ce qu'il contient une quantité efficace d'au moins un composé de formule générale I selon la revendication 1 ou d'un sel d'un tel composé, avec des produits auxiliaires de formulation.

10. Produit fongicide selon la revendication 9, caractérisé en ce qu'il contient une quantité efficace d'au moins un composé de formule générale Ia selon la revendication 4 avec des produits auxiliaires de formulation.

11. Produit fongicide selon la revendication 9, caractérisé en ce qu'il contient une quantité efficace d'alpha-éthynyl-alpha-(2,4-dichloro-alpha-méthylthiobenzyl)-3-pyridylméthanol avec des produits auxiliaires de formulation.

12. Produit fongicide selon la revendication 9, caractérisé en ce qu'il contient une quantité efficace d'alpha-éthynyl-alpha-[2,4-dichloro-alpha-(2-propynyl)-benzyl]-3-pyridylméthanol avec des produits auxiliaires de formulation.

13. Produit fongicide selon la revendication 9, caractérisé en ce qu'il contient une quantité efficace d'alpha-(2,4-dichloro-alpha-méthylbenzyl)-alpha-[(Z)-1-penténył]-3-pyridylméthanol avec des produits auxiliaires de formulation.

14. Procédé de préparation des composés de formule générale I selon la revendication 1 et de leurs sels formés par addition avec des acides, caractérisé en ce que :

a) pour préparer les composés de formule I dans laquelle $R^2$ représente un groupe hydroxy et $R^4$ un groupe 3-pyridyle, 2-pyrazinyle ou 5-pyrimidinyle et $R^3$ a les significations indiquées ci-dessus, à l'exception de C≡CBr ou de C≡CI, on fait réagir une cétone de formule générale

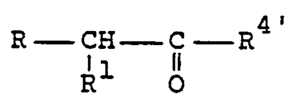

(II)

dans laquelle

$R$ et $R^1$ ont les significations indiquées ci-dessus, et

$R^{4'}$ représente un groupe 3-pyridyle, 2-pyrazinyle ou 5-pyrimidinyle,

avec un composé de formule générale

$$(R^{3'})_nX \qquad \text{(III)}$$

dans laquelle

$R^{3'}$ a les significations de $R^3$, à l'exception de C≡CBr ou C≡CI et

X représente un métal alcalin, un métal alcalino-terreux ou MgHal,

n est la valence de X, et

Hal représente un halogène,

b) pour préparer les composés de formule I dans laquelle $R^2$ représente un groupe hydroxy, $R^3$ le groupe C≡CBr ou C≡CI et $R^4$ un groupe 3-pyridyle, 2-pyrazinyle ou 5-pyrimidinyle, on traite un dérivé de l'éthyne de formule générale

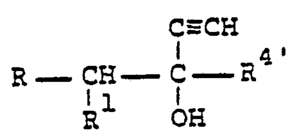

(I')

dans laquelle R, $R^1$ et $R^{4'}$ ont les significations indiquées ci-dessus, par un agent bromant ou iodant respectivement,

c) pour préparer les composés de formule I dans laquelle $R^2$ représente le chlore ou le brome et $R^4$ un groupe 3-pyridyle, 2-pyrazinyle ou 5-pyrimidinyle, on traite un alcool de formule générale

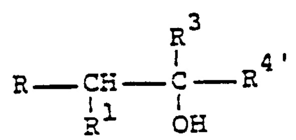

(I")

dans laquelle R, $R^1$, $R^3$ et $R^{4'}$ ont les significations indiquées ci-dessus, par un agent chlorant ou bromant respectivement,

d) pour préparer les composés de formule I dans laquelle $R^3$ représente un groupe 1-alcényle en C 2-

C 6 et $R^4$ un groupe 3-pyridyle, 2-pyrazinyle ou 5-pyrimidinyle, on réduit un dérivé d'alcyne de formule générale

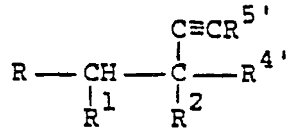

(I''')

dans laquelle

R, $R^1$, $R^2$ et $R^{4'}$ ont les significations indiquées ci-dessus et

$R^{5'}$ représente l'hydrogène ou un groupe alkyle en C 1-C 4, ou bien

e) pour préparer les composés de formule I dans laquelle $R^4$ représente un groupe 1-oxyde de 3-pyridyle, 1-oxyde de 2-pyrazinyle, 4-oxyde de 2-pyrazinyle, 1,4-dioxyde de 2-pyrazinyle, 1-oxyde de 5-pyrimidinyle ou 1,3-dioxyde de 5-pyrimidinyle,

on soumet à N-oxydation respectivement un dérivé de pyridine, de pyrazine ou de pyrimidine de formule générale

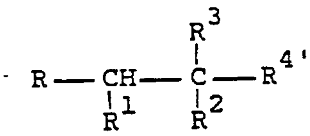

(I'''')

dans laquelle R, $R^1$, $R^2$, $R^3$ et $R^{4'}$ ont les significations indiquées ci-dessus, et si on le désire, on convertit un composé obtenu répondant à la formule I, par réaction avec un acide, en le sel correspondant formé par addition.

15. Procédé pour combattre les fungis dans l'agriculture et l'horticulture, caractérisé en ce que l'on traite le bien à protéger par une quantité efficace d'un composé selon l'une des revendications 1 à 6 ou d'un produit selon l'une des revendications 9 à 12.

16. Procédé pour combattre les fungis dans l'agriculture et l'horticulture, caractérisé en ce que l'on traite le bien à protéger par une quantité efficace d'un composé selon la revendication 7 ou d'un produit selon la revendication 13.

17. Utilisation d'un composé selon l'une des revendications 1 à 6, ou d'un produit selon l'une des revendications 9 à 12, dans la lutte contre les fungis dans l'agriculture et l'horticulture.

18. Utilisation d'un composé selon la revendication 7 ou d'un produit selon la revendication 13 dans la lutte contre les fungis dans l'agriculture et l'horticulture.

**Revendications** (pour l'Etat contractant AT)

1. Produit fongicide caractérisé en ce qu'il contient une quantité efficace d'au moins un composé de formule générale

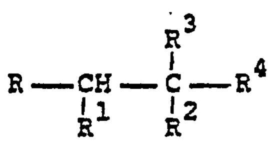

(I)

dans laquelle

R représente un groupe phényle mono-, di- ou tri-substitué, les substituants consistant en 1 à 3 atomes d'halogènes et/ou 1 ou 2 groupes alkyle en C 1-C 3 et/ou 1 ou 2 groupes alcoxy en C 1-C 3 et/ou 1 ou 2 groupes trifluorométhyle,

$R^1$ représente un groupe alkyle en C 1-C 6, alcényle en C 3-C 6, alcynyle en C 3-C 6, alkylthio en C 1-C 6, alcénylthio en C 3-C 6 ou alcynylthio en C 3-C 6, éventuellement substitué par un groupe alcoxy en C 1-C 4 ; ou bien un groupe arylthio éventuellement mono-, di- ou tri-substitué, les substituants éventuels consistant en 1 à 3 atomes d'halogènes et/ou un ou deux groupes alkyle en C 1-C 3 et/ou un ou deux groupes alcoxy en C 1-C 3 et/ou un groupe nitro,

$R^2$ représente un groupe hydroxy, le chlore ou le brome,

$R^3$ représente un groupe alcényle en C 2-C 6 ; un groupe aryle ou aryl-alkyle en C 1-C 3 éventuellement mono-, di- ou tri-substitué dans le noyau aryle, les substituants éventuels consistant en 1 à 3 atomes d'halogènes et/ou un ou deux groupes alkyle en C 1-C 3 et/ou un ou deux groupes alcoxy en C 1-C 3 et/ou un groupe nitro ; ou un groupe $C{\equiv}CR^5$,

$R^4$ représente un groupe 3-pyridyle, 1-oxyde de 3-pyridyle, 2-pyrazinyle, 1-oxyde de 2-pyrazinyle, 4-

oxyde de 2-pyrazinyle, 1,4-dioxyde de 2-pyrazinyle, 5-pyrimidinyle, 1-oxyde de 5-pyrimidinyle ou 1,3-dioxyde de 5-pyrimidinyle, et

$R^5$ représente l'hydrogène, un groupe alkyle en C 1-C 10 éventuellement substitué par un ou deux groupes alcoxy en C 1-C 3, un atome de chlore ou un atome de brome ; un groupe vinyle ; un groupe alcoxy en C 1-C 6 ; le brome ; l'iode ; ou un groupe aryle éventuellement mono-, di- ou tri-substitué, les substituants éventuels consistant en 1 à 3 atomes d'halogènes et/ou un ou deux groupes alkyle en C 1-C 3 et/ou un ou deux groupes alcoxy en C 1-C 3,

ou d'un sel d'un tel composé formé par addition avec un acide, et des produits auxiliaires de formulation.

2. Produit fongicide selon la revendication 1, dans lequel R représente un groupe 2,4-dichlorophényle.

3. Produit fongicide selon la revendication 1 ou 2, dans lequel $R^4$ représente un groupe 3-pyridyle, 2-pyrazinyle ou 5-pyrimidinyle.

4. Produit fongicide selon la revendication 1, caractérisé en ce qu'il contient une quantité efficace d'au moins un composé de formule

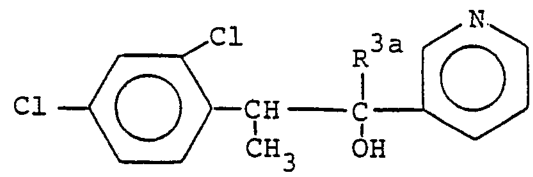

(Ia)

dans laquelle $R^{3a}$ représente un groupe vinyle, 1-propène-1-yle, isopropényle, éthynyle, 1-pentynyle, éthoxyéthynyle ou iodoéthynyle, avec des produits auxiliaires de formulation.

5. Produit fongicide selon la revendication 1, caractérisé en ce qu'il contient une quantité efficace d'alpha-éthynyl-alpha-(2,4-dichloro-alpha-méthylthiobenzyl)-3-pyridylméthanol avec des produits auxiliaires de formulation.

6. Produit fongicide selon la revendication 1, caractérisé en ce qu'il contient une quantité efficace d'alpha-éthynyl-alpha-[2,4-dichloro-alpha-(2-propynyl)-benzyl]-3-pyridylméthanol avec des produits auxiliaires de formulation.

7. Produit fongicide selon la revendication 1, caractérisé en ce qu'il contient une quantité efficace d'alpha-(2,4-dichloro-alpha-méthylbenzyl)-alpha-[(Z)-1-pentényl]-3-pyridylméthanol avec des produits auxiliaires de formulation.

8. Procédé de préparation des composés de formule générale I selon la revendication 1, et de leurs sels formés par addition avec des acides, caractérisé en ce que :

a) pour préparer les composés de formule I dans laquelle $R^2$ représente un groupe hydroxy et $R^4$ un groupe 3-pyridyle, 2-pyrazinyle ou 5-pyrimidinyle et $R^3$ a les significations indiquées ci-dessus, à l'exception de C≡CBr ou de C≡CI, on fait réagir une cétone de formule générale

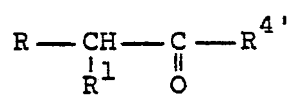

(II)

dans laquelle

R et $R^1$ ont les significations indiquées ci-dessus, et $R^{4'}$ représente un groupe 3-pyridyle, 2-pyrazinyle ou 5-pyrimidinyle,

avec un composé de formule générale

$$(R^{3'})_nX \qquad (III)$$

dans laquelle

$R^{3'}$ a les significations de $R^3$, à l'exception de C≡CBr ou C≡CI et

X représente un métal alcalin, un métal alcalino-terreux ou MgHal,

n est la valence de X, et

Hal représente un halogène,

b) pour préparer les composés de formule I dans laquelle $R^2$ représente un groupe hydroxy, $R^3$ le groupe C≡CBr ou C≡CI et $R^4$ un groupe 3-pyridyle, 2-pyrazinyle ou 5-pyrimidinyle, on traite un dérivé de l'éthyne de formule générale

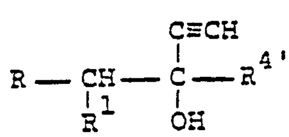

(I')

dans laquelle R, $R^1$ et $R^{4'}$ ont les significations indiquées ci-dessus, par un agent bromant ou iodant respectivement,

c) pour préparer les composés de formule I dans laquelle $R^2$ représente le chlore ou le brome et $R^4$ un groupe 3-pyridyle, 2-pyrazinyle ou 5-pyrimidinyle, on traite un alcool de formule générale

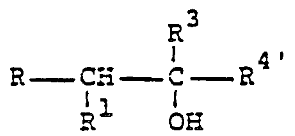

(I'')

dans laquelle R, $R^1$, $R^3$ et $R^{4'}$ ont les significations indiquées ci-dessus, par un agent chlorant ou bromant respectivement,

d) pour préparer les composés de formule I dans laquelle $R^3$ représente un groupe 1-alcényle en C 2-C 6 et $R^4$ un groupe 3-pyridyle, 2-pyrazinyle ou 5-pyrimidinyle,
on réduit un dérivé d'alcyne de formule générale

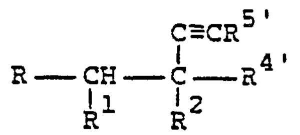

(I''')

dans laquelle

R, $R^1$, $R^2$ et $R^{4'}$ ont les significations indiquées ci-dessus et

$R^{5'}$ représente l'hydrogène ou un groupe alkyle en C 1-C 4, ou bien

e) pour préparer les composés de formule I dans laquelle $R^4$ représente un groupe 1-oxyde de 3-pyridyle, 1-oxyde de 2-pyrazinyle, 4-oxyde de 2-pyrazinyle, 1,4-dioxyde de 2-pyrazinyle, 1-oxyde de 5-pyrimidinyle ou 1;3-dioxyde de 5-pyrimidinyle,
on soumet à N-oxydation respectivement un dérivé de pyridine, de pyrazine ou de pyrimidine de formule générale

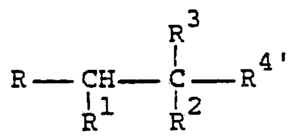

(I'''')

dans laquelle R, $R^1$, $R^2$, $R^3$ et $R^{4'}$ ont les significations indiquées ci-dessus et si on le désire, on convertit un composé obtenu répondant à la formule I, par réaction avec un acide, en le sel correspondant formé par addition.

9. Procédé de préparation d'un produit fongicide caractérisé en ce que l'on mélange au moins un des composés mentionnés dans la revendication 1 avec des produits auxiliaires de formulation.

10. Procédé pour combattre les fungis dans l'agriculture et l'horticulture, caractérisé en ce que l'on traite le bien à protéger par une quantité efficace d'un composé ou d'un produit selon l'une des revendications 1 à 6.

11. Procédé pour combattre les fungis dans l'agriculture et l'horticulture, caractérisé en ce que l'on traite le bien à protéger par une quantité efficace d'un composé ou d'un produit selon la revendication 7.

12. Utilisation d'un composé ou d'un produit selon l'une des revendications 1 à 6 dans la lutte contre les fongis dans l'agriculture et l'horticulture.

13. Utilisation d'un composé ou d'un produit selon la revendication 7 dans la lutte contre les fungis dans l'agriculture et l'horticulture.